# EUROPEAN PATENT SPECIFICATION

(11) **EP 4 457 212 B1**
(45) Date of publication and mention of the grant of the patent: **10.12.2025**
(21) Application number: 22844176.2
(22) Date of filing: 23.12.2022
(51) Int. Cl.: C07C 381/10

(54) **METHOD FOR PREPARING IONIC COMPOUNDS**
VERFAHREN ZUR HERSTELLUNG VON IONISCHEN VERBINDUNGEN
PROCÉDÉS DE PRÉPARATION DE COMPOSÉS IONIQUES

(30) Priority: 27.12.2021 EP 21383222
(43) Date of publication of application: 06.11.2024
(73) Proprietor: Fundación Centro de Investigación Cooperativa de Energías Alternativas, CIC Energigune Fundazioa, 01510 Vitoria-Gasteiz, Álava (ES)
(72) Inventor: ARMAND, Michel, 01510 Vitoria-Gasteiz, Álava (ES); ZHANG, Heng, 01510 Vitoria-Gasteiz, Álava (ES); GARCÍA, Lorena, 01510 Vitoria-Gasteiz, Álava (ES); MARTÍNEZ-IBÁÑEZ, María, 01510 Vitoria-Gasteiz, Álava (ES); SÁNCHEZ DÍEZ, Eduardo, 01510 Vitoria-Gasteiz, Álava (ES)
(74) Representative: ABG Intellectual Property Law, S.L.
(86) International application number: PCT/EP2022/087831
(87) International publication number: WO 2023/126377

(56) References cited:
- US-A1- 2019 165 417
- US-B1- 6 340 716
- R.Y. GARLYAUSKAYTE, ET AL.: "Synthesis of new organic super acids - N-(trifluoromethylsulfonyl)imino derivatives of trifluoromethanesulfonic acid and bis(trifluoromethylsulfonyl)imide", ORGANIC AND BIOMOLECULAR CHEMISTRY, vol. 3, no. 12, 11 May 2005 (2005-05-11), Royal Society of Chemistry, Cambridge, GB, pages 2239 - 2243, XP055573162, ISSN: 1477-0520, DOI: 10.1039/b503286p
- T.P. SMYTH, ET AL.: "S-Aminosulfeniminopenicillins: multimode beta-lactamase inhibitors and template structures for penicillin-based beta-lactamase substrates as prodrugs", JOURNAL OF ORGANIC CHEMISTRY, vol. 63, no. 22, 9 October 1998 (1998-10-09), American Chemical Society, Washington, DC, US, pages 7600 - 7618, XP002204183, ISSN: 0022-3263, DOI: 10.1021/JO970737F
- H. BAO, ET AL.: "Catalytic enantioselective allylic Amination of unactivated terminal olefins via an ene reaction/[2,3]-rearrangement", JOURNAL OF THE AMERICAN CHEMICAL SOCIETY, vol. 134, no. 45, 29 October 2012 (2012-10-29), American Chemical Society, Washington, DC, US, pages 18495 - 18498, XP055930010, ISSN: 0002-7863, DOI: 10.1021/ja307851b
- H. BAO, ET AL.: "Supporting information for: Catalytic enantioselective allylic amination of unactivated terminal olefins via an ene reaction/[2,3]-rearrangement", JOURNAL OF THE AMERICAN CHEMICAL SOCIETY, vol. 134, no. 45, 29 October 2012 (2012-10-29), American Chemical Society, Washington, DC, US, pages S1 - S65, XP055930016, ISSN: 0002-7863, DOI: 10.1021/ja307851b

## Description

### FIELD OF THE INVENTION

This invention relates to a method for preparing ionic compounds which may find application in various fields such as electrochemistry, catalysis, materials chemistry, among which it is worth mentioning the field of rechargeable batteries, especially lithium and sodium batteries. This invention also relates to a synthetic intermediate in the route leading to said ionic compounds.

### BACKGROUND

Electrolytes enable, according to their ionic conductivity, the movement of ions from the cathode to the anode on charge and in reverse on discharge. The electrolyte of a battery may consist of soluble salts, acids or other bases in liquid, gelled and dry formats. For example, it is well known that the salts of strong acids such as HClO₄, HBF₄, HPF₆ and HR_{F}SO₃ (R_{F} = perfluoroalkyl radical) have electrochemical properties. The "superacids" obtained by adding a Lewis acid such as SbF₅ to the abovementioned compounds are also known. However, these compounds are not stable other than in protonated form and in non-solvating media such as aliphatic hydrocarbons. The salts are unstable in the usual polar solvents.

Electrolytes can also be polymeric. The conductivity of polymer electrolytes, which is intrinsically low as they are primarily organic and contain ions only adventitiously in very small concentrations, may be increased by the addition of certain amounts of ionic compounds (or salts).

In particular, ionic compounds such as perfluoroalkylsulfonimide derivatives M[R_{F}SO₂NSO₂R_{F}] (R_{F}=perfluoroalkyl) have recently raised more attention across various chemical fields. They have advantageous stability properties in protonated form (M is H) or in the form of salts (M is commonly a metal) and are used as solutes in electrochemistry and as catalysts. However, it is challenging to give these salts all desirable properties required for their applications, in particular in terms of properties such as acidity, dissociation ability or solubility. Among known M[R_{F}SO₂NSO₂R_{F}] derivatives, lithium bis(trifluoromethanesulfonyl)imide) (Li[(CF₃SO₂)₂N], also abbreviated as LiTFSI, has been studied in recent years as a potential replacement of lithium hexafluorophosphate (LiPF₆), which currently dominates the landscape of ionic compounds for non-aqueous liquid electrolytes. LiTFSI is more chemically and thermally stable when compared to LiPF₆ and has already found application as conducting salt in aqueous electrolytes of lithium batteries having lithium metal as anode.

H. Zhang et al. (J. Power Sources 2015, 296, 142-149) report the physiochemical and electrochemical characterization of neat LisTFSI, namely [CF₃-S(O)₂-N⁻-S(O)(CF₃)-N-S(O)₂-CF₃] , and its carbonated-based liquid electrolytes compared to LiPF₆, LiTFSI and (Li[(C₂F₅SO₂)₂N] (abbreviated as LiBETI). The liquid electrolyte is formed from LisTFSI in a mixture of ethylene carbonate (EC) and ethyl-methyl-carbonate (EMC) with small amounts of water. Despite the higher dissociation ability of LisTFSI, compared to the parent compound LiTFSI, its ionic conductivity was found to be smaller than that of LiTFSI, which is attributed to the larger anionic volume of [sTFSI]⁻ compared to that of [TFSI]⁻.

H. Zhang et al. (ChemElectroChem 2021, 8, 1322-1328) disclose a LisTFSI/PEO (poly(ethylene oxide)) membrane that is characterized by a slightly superior Li⁺ ion only conductivity but again considerably lower total ionic conductivity compared to those of a standard LiTFSI/PEO solid electrolyte.

Based on the higher dissociation ability of LisTFSI, a higher Li⁺ mobility was expected, however these data reinforce the idea that the structural optimization of a conductive salt, especially in terms of its total conductivity, is not a trivial task.

At the same time, it is concerning that the routes to new and potentially useful perfluorosulfonimide derivatives are based on time-consuming, wasteful and scope-limited synthetic steps. The first synthesis of K[sTFSI] (the corresponding potassium salt of the compound disclosed in H. Zhang et al., ChemElectroChem 2021, 8, 1322 - 1328) was reported more than a decade ago by R. Y. Garlyauskayte et al. (Org. Biomol. Chem. 2005, 3, 2239-2243) and it is still adopted nowadays to access ionic compounds of formula M[sTFSI] salts (M is a cation), which offer a fertile ground for optimization of electrolytes in secondary cell and battery applications. R. Y. Garlyauskayte et al. describe two sulfoximidoyl fluoride intermediates, PhS(O)(NSO₂CF₃)F (1) and CF₃S(O)(NSO₂CF₃)F (2), which act as synthons for the synthesis of salts of perfluorosulfonimide derivatives. However, PhS(O)(NSO₂CF₃)F (1) only afforded symmetric perfluorosulfonimide dimers by reaction with Li₃N in the presence of dimethylaminopyridine as catalyst and subsequent treatment with KCl. The inherent limitation of this method through intermediate (1) is that unsymmetric perfluorosulfonimides cannot be obtained. Using the same method, intermediate (2) gave very low product yields and the isolation was unsuccessful. On the other hand, by changing the reaction conditions, the intermediate CF₃S(O)(NSO₂CF₃)F (2) was converted to two perfluorosulfonimide salts via a one-pot two-steps sequence involving a) reaction with amides in presence of pyridine in a methylene chloride solution and b) exchange of the pyridinium cation with a potassium cation by either use of an ion-exchange resin (such as Dowex 50WX8-200, K⁺ form) or reaction with K₂CO₃ in methylene chloride. Even though the products are symmetric, in principle the same route could afford unsymmetric salts. Despite its higher modulability, it is apparent that the synthetic efficiency of the second method is affected by the electronic nature of the starting materials as intermediate (1) only led to a 25% isolated product yield. Additionally, the full route to perfluorosulfonimide salts via the intermediate CF₃S(O)(NSO₂CF₃)F (2) (shown below) displays a number of drawbacks such as a long sequence (5 steps starting from the precursor CF₃SO₂NH₂), long overall reaction time (ca. 12 hours), use of environmentallyunfriendly reagents (CF₃SiMe₃ is prepared from bromotrifluoromethane, a well-known depleter of the stratospheric ozone layer), poor atom-economy (excess of CsF generates fluorine waste), cryogenic conditions, narrow nucleophile scope (the anionic S(IV) intermediate only reacts with F⁻) and the complex purification of intermediates (1) and (2) which are moisture sensitive.

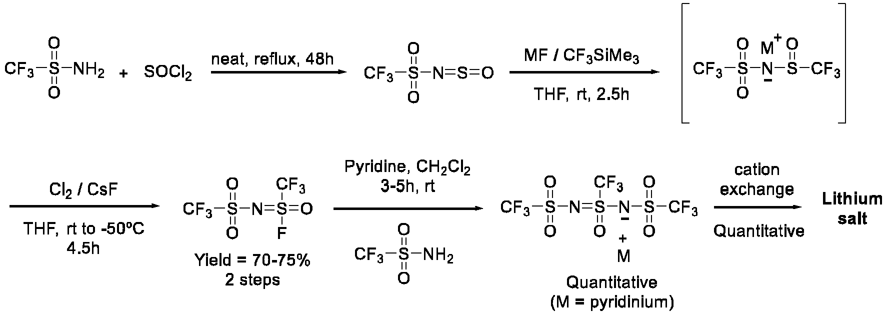

US6340716B1 describes a wide range of ionic compounds that are useful for the production of ion conducting materials or electrolytes, as catalysts and for doping polymers, though the general synthetic procedures do not depart from what is taught by R. Y. Garlyauskayte et al.

This document reports that a compound of formula [R¹SO₂N-S*(=O)(R²)=NSO₂R³][M] (such as, i.e. LisTFSI) can be obtained by reacting a salt [[R¹SO₂N-S*(=O)(R²)][M] with a halogenating agent, to give the intermediate [R¹SO₂N-S*(=O)(R²)(X)] (X being a halogen), which is structurally analogous to intermediate (2) described earlier. Said precursor is then condensed with a sulfonamide R³SO₂NH₂ in the presence of a base or with the metallic derivatives of the sulfonamide, such as R³SO₂NLi₂ or R³SO₂NNa₂. The desired cation for the final compound is obtained by standard ion-exchange processes.

US 2019/165417 A1 discloses compounds of formula [R¹-S(=O)2-N=S(=O)(R³)-N-S(=O)2-R²] [M], for example where R¹, R² and R³ are F and M is Li, and their preparation by the reaction of F-S(=O)₂-N-S(=O)₂-F with itself.

In view of the drawbacks associated to a route involving an intermediate of formula [R¹SO₂N-S*(=O)(R²)(X)] as detailed above, more sustainable procedures to prepare sulfonimide salts should be sought after, in particular with the aim of finding new ionic compounds for use in solid electrolytes that possess high ionic conductivity.

Thus, the aim of the present invention is the provision of a less wasteful, step-economical and facile method to prepare ionic compounds with a sulfonimide backbone which not only display a high delocalization of the anionic charge and improved dissociation ability, but can be also comprised in solid-state electrolyte to improve the total ionic conductivity.

### BRIEF DESCRIPTION OF THE INVENTION

The inventors have devised a new method to prepare ionic compounds (more conveniently called "salts", from hereinafter) of formula I as defined elsewhere herein, said compounds feature high delocalization of the negative charge in the anion and can patently increase the total ionic conductivity of a solid electrolyte in which they can be comprised. The anion of salt I involves an S-N-S-N-S core where sulfur atoms are hexavalent and the negative charge is delocalized between the two nitrogen atoms and the five oxygen atoms linked to the sulfur atoms; and, specifically, a group (E) and two groups R¹ and R² are directly attached to the S-N-S-N-S core. It has unexpectedly been found that salts of this type lead to improved solid electrolytes that are more conductive than reported solid electrolyte comprising analogous salts with no fluorine substituents linked to any of the sulfur atoms.

The method described herein is performed in one-pot and displays several advantages compared to prior art, among these: higher step-and atom-economy, milder conditions, as well as the ability to synthesize a broad range of salts having high negative charge delocalization as mentioned above.

Therefore, in a first aspect, the present invention refers to a method to obtain a salt of formula I: 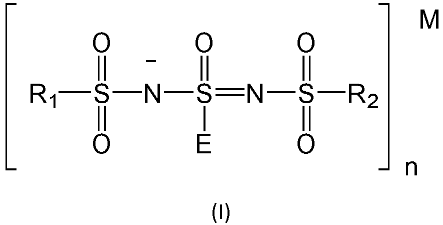 wherein:
- E is a group selected from F; Cl; Br; I; -N(R₄)(R₅), wherein R₄ and R₅ are independently selected from H, an alkyl and an ethylene oxide chain having from 1 to 5 repeating units of ethylene oxide; an alkyl optionally substituted with at least one F, an alkenyl, an aryl, -N(R₆)(R₇) or O(R₈), wherein R₆, R₇ and R₈ are independently selected from H and alkyl; and an arylalkenyl;
- R₁ and R₂ are independently selected from:
- halogen;
- -Y, wherein Y represents:
   - an organic radical selected from alkyl, alkenyl, alkynyl, aryl, alkylaryl, arylalkyl, alkylene oxide or alkylene imine, optionally substituted with at least a substituent selected from the group consisting of F, Cl, Br, I, -CN, -OR', -SR', -NR'₂, wherein R' is H, alkyl, alkylene oxide or alkylene imine; or
   - a polymeric group comprising repeating units selected from alkylene oxide, alkylene imine, acrylate, maleimide, phosphazene, siloxane, vinyl alcohol, vinyl amine or mixtures thereof; and
- -OY, -SY, -NY₂, wherein Y represents:
   - H or an organic radical selected from alkyl, alkenyl, alkynyl, aryl, alkylaryl, arylalkyl, alkylene oxide or alkylene imine, optionally substituted with at least a substituent selected from the group consisting of F, Cl, Br, I, -CN, -OR', -SR', -NR'₂, wherein R' is H, alkyl, alkylene oxide or alkylene imine; or
   - a polymeric group comprising repeating units selected from alkylene oxide, alkylene imine, acrylate, maleimide, phosphazene, siloxane, vinyl alcohol, vinyl amine or mixtures thereof;
   wherein each of the two specific substituents Y at NY₂ and R' at NR'₂ is selected independently from the other,
- M is:
   - a monovalent, divalent or trivalent cation, selected from ions of alkali metals, of alkaline earth metals, of transition metals and of rare-earth metals;
   - an organic onium or polyonium cation; or
   - an organometallic cation;
   - n is an integer positive number;
said method comprising the steps of:
a) reacting a N-(sulfinyl)sulfonamide of formula (II): 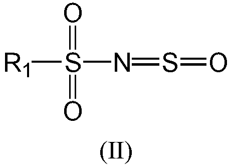 wherein R¹ is as defined above;
   with a sulphonamide salt of formula (III): 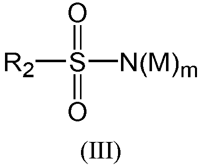 wherein
      - R₂ is as defined above,
      - M is a proton, or a monovalent or divalent cation selected from ions of alkali metals, of alkaline earth metals, of transition metals, of rare-earth metals, an organic onium cation and an organometallic cation;
      - m is 1 if M is a divalent cation, or 2 if M is a proton or a monovalent cation;
         provided that when M is a proton, step a) is carried out in presence of a base,
   to obtain a dianionic intermediate of formula (IV): 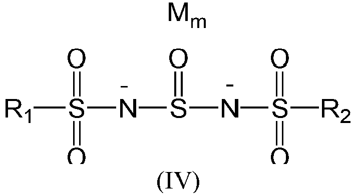 wherein
      - R₁, R₂ are as defined above;
      - M is a monovalent or divalent cation, selected from ions of alkali metals, of alkaline earth metals, of transition metals, of rare-earth metals, an organic onium cation and an organometallic cation; and
      - m is 1 if M is a divalent cation, or 2 if M is a monovalent cation;
b) oxidizing the compound of formula (IV) with a reagent selected from a fluorination reagent, a chlorination reagent, a bromination reagent, a iodination reagent, an alkylation reagent, and an amination reagent,
   to give a salt of formula (I), wherein:
   - R₁, R₂ and E are as defined above;
   - M is a monovalent or a divalent cation, selected from ions of alkali metals, of alkaline earth metals, of transition metals, of rare-earth metals, an organic onium and an organometallic cation; and
   - n is 1 or 2 and corresponds to the valency of M; and
c) carrying out a cation exchange reaction to replace the monovalent or divalent cation M of the salt of formula (I) resulting from step b) by a trivalent or polyonium cation if a salt of formula (I) where M is a trivalent cation or a polyonium cation is to be obtained.

The method can be illustrated according to the general scheme below:

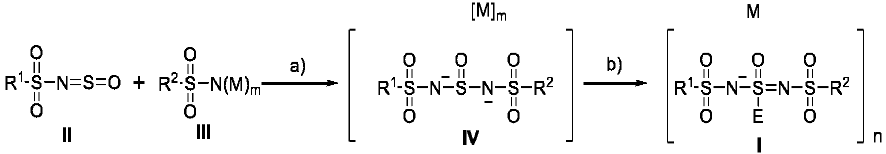

The process can also include an optional step wherein a fluoride radical at E position in the general formula (I) is replaced by a different radical E, namely by a C₁-C₁₆ alkyl radical or an amine radical N(R₄)(R₅) by nucleophilic substitution reactions.

Furthermore, an additional cation exchange step may be performed where the cation M is replaced by a different cation M'. In the particular case wherein it is desired to obtain a salt of formula (I) wherein the cation is a mono- or di-valent cation different from that in compounds III and IV or is a trivalent, such as Al³⁺, or a polyonium cation, the salt of formula (I) resulting from step b) is subjected to said cation exchange step. A skilled person would know how to perform such additional step, generally by reacting the salt containing the cation M with a suitable salt containing the other cation M' or directly with a M' in a solvent, adjusting the amounts of the reacting salts so that the charges of the anion and the cation are balanced, and letting the exchange reaction go to completion.

In another aspect, the invention refers to a dianionic compound of formula (IV): 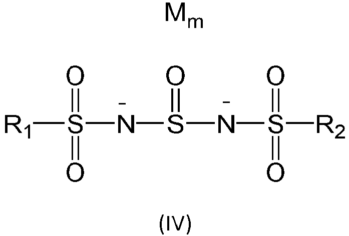 wherein
- R₁, R₂ are defined as in the method above;
- M is a monovalent or divalent cation, selected from ions of alkali metals, of alkaline earth metals, of transition metals, of rare-earth metals, an organic onium cation and an organometallic cation; and
- m is 1 if M is a divalent cation, or 2 if M is a monovalent cation;

### DETAILED DESCRIPTION OF THE INVENTION

The present invention refers to a method to obtain a salt of formula **I:** 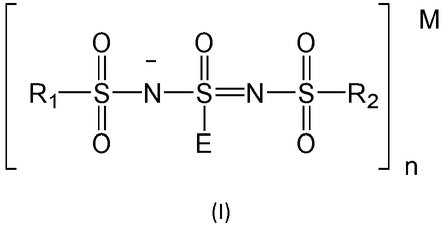 wherein:
- E is a group selected from F; Cl; Br; I; N(R₄)(R₅), wherein R₄ and R₅ are independently selected from H, a alkyl and an ethylene oxide chain having from 1 to 5 repeating units of ethylene oxide; an alkyl optionally substituted with at least one F, an alkenyl, an aryl, -N(R₆)(R₇) or O(R₈), wherein R₆, R₇ and R₈ are independently selected from H and alkyl; and an arylalkenyl;
- R₁ and R₂ are independently selected from
- halogen;
- -Y, wherein Y represents:
   - an organic radical selected from alkyl, alkenyl, alkynyl, aryl, alkylaryl, arylalkyl, alkylene oxide or alkylene imine, optionally substituted with at least a substituent selected from the group consisting of F, Cl, Br, I, -CN, -OR', -SR', -NR'₂, wherein R' is H, alkyl, alkylene oxide or alkylene imine; or
   - a polymeric group comprising repeating units selected from alkylene oxide, alkylene imine, acrylate, maleimide, phosphazene, siloxane, vinyl alcohol, vinyl amine or mixtures thereof; and
- -OY, -SY, -NY₂, wherein Y represents:
   - H or an organic radical selected from alkyl, alkenyl, alkynyl, aryl, alkylaryl, arylalkyl, alkylene oxide or alkylene imine, optionally substituted with at least a substituent selected from the group consisting of F, Cl, Br, I, -CN, -OR', -SR', -NR'₂, wherein R' is H, alkyl, alkylene oxide or alkylene imine; or
   - a polymeric group comprising repeating units selected from alkylene oxide, alkylene imine, acrylate, maleimide, phosphazene, siloxane, vinyl alcohol, vinyl amine or mixtures thereof;
   wherein each of the two specific substituents Y at NY₂ and R' at NR'₂ is selected independently from the other,
- M is:
   - a monovalent, divalent or trivalent cation, selected from ions of alkali metals, of alkaline earth metals, of transition metals and of rare-earth metals;
   - an organic onium or polyonium cation; or
   - an organometallic cation;
- n is an integer positive number which corresponds to the valency of M;
said method comprising the steps of:
a) reacting a N-(sulfinyl)sulfonamide of formula (II): 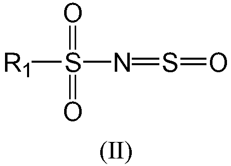 wherein R¹ is as defined above;
   with a sulphonamide salt of formula (III): 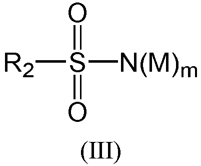 wherein
      - R₂ is as defined above,
      - M is a proton, or a monovalent or a divalent cation, selected from ions of alkali metals, of alkaline earth metals, of transition metals, of rare-earth metals, an organic onium cation, and an organometallic cation;
      - m is 1 if M is a divalent cation, or 2 if M is a proton or a monovalent cation;
         provided that when M is a proton, step a) is carried out in presence of a base,
   to obtain a dianionic intermediate compound of formula (IV): 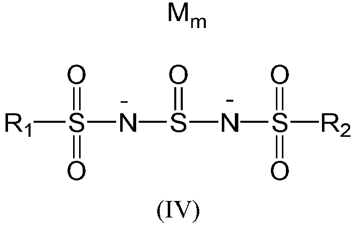 wherein
      - R₁, R₂ are as defined above;
      - M is a monovalent or a divalent cation, selected from ions of alkali metals, of alkaline earth metals, of transition metals, of rare-earth metals, an organic onium cation, and an organometallic cation; and
      - m is 1 if M is a divalent cation, or 2 if M is a monovalent cation;
b) oxidazing the compound of formula (IV) with a reagent selected from a fluorination reagent, a chlorination reagent, a bromination reagent, a iodination reagent, an alkylation reagent and an amination reagent,
   to give a salt of formula (I), wherein:
   - R₁, R₂ and E are as defined above;
   - M is a monovalent or a divalent cation, selected from ions of alkali metals, of alkaline earth metals, of transition metals, of rare-earth metals, an organic onium cation, and an organometallic cation; and
   - n is 1 or 2 and corresponds to the valency of M; and
c) carrying out a cation exchange reaction to replace the monovalent or divalent cation M of the salt of formula (I) resulting from step b) by a trivalent or a polyonium cation if a salt of formula (I) where M is a trivalent cation or a polyonium cation is to be obtained.

The subscripts "m" and "n" are integer positive numbers equal to 1, 2 or 3 and refer to the number of cations or anions needed to neutralize the charge of the corresponding counterion, respectively, so that the ionic compound is overall neutral. Preferably, m is 1 or 2. Also preferably n is 1 or 2.

The term "alkyl radical" refers to an alkane-derived radical which is bound to the rest of the molecule through a single bond. It may be linear or branched. It preferably comprises from 1 to 16 ("C₁-C₁₆ alkyl"), preferably from 1 to 8 ("C₁-C₈ alkyl"), even more preferably from 1 to 4 ("C₁-C₄ alkyl") carbon atoms. Illustrative examples of alkyl groups include methyl, ethyl, n-propyl, i-propyl, n-butyl, i-butyl, t-butyl, pentyl, hexyl, heptyl, octyl.

The term "aryl radical" refers to an aromatic group, preferably having between 6 and 10 carbon atoms ("C₆-C₁₀ aryl"), which may comprise 1 aromatic ring or 2 aromatic rings fused to one another. Illustrative examples of aryl groups include phenyl, naphthyl or indenyl. Preferably, it is phenyl.

The term "alkenyl radical" refers to an alkene-derived radical which is bound to the rest of the molecule through a single bond. It may be linear or branched. It preferably comprises from 2 to 16 ("C₂-C₁₆ alkenyl"), preferably from 2 to 8 ("C₂-C₈ alkenyl"), even more preferably from 2 to 4 ("C₂-C₄ alkenyl") carbon atoms.

The term "alkynyl radical" refers to an alkyne derived radical which is bound to the rest of the molecule through a single bond. It may be linear or branched. It preferably comprises from 2 to 16 ("C₂-C₁₆ alkynyl"), preferably from 2 to 8 ("C₂-C₈ alkynyl"), even more preferably from 2 to 4 ("C₂-C₄ alkynyl") carbon atoms.

The term "alkylaryl radical" refers to a radical derived from a group comprising an alkyl as defined above bearing at least an aryl group as described above along the alkyl chain and which is bound to the rest of the molecule through the alkyl group.

The term "arylalkyl radical" refers to a radical derived from a group comprising an alkyl as defined above bearing at least an aryl group as described above along the alkyl chain and which is bound to the rest of the molecule through the aryl group.

The term "alkylene oxide radical" refers to a radical derived from a saturated aliphatic chain containing alkylene oxide units which is bound to rest of the molecule through a single bond. It may be linear or branched. The alkylene portion refers to an alkane-derived diradical. The term "alkylene" is generally employed as meaning non-terminal alkyl moieties. The alkylene oxide radical can be depicted through general formula -(alkylene-O)ₓRₐₒ, wherein x is from 1 to 5, more preferably 1 or 2; the alkylene portion comprises from 1 to 16 ("C₁-C₁₆ alkylene"), preferably from 1 to 8 ("C₁-C₈ alkylene"), even more preferably from 1 to 4 ("C₁-C₄ alkylene") carbon atoms; and Rₐₒ is H or alkyl as described above, preferably H or methyl, more preferably H. Preferably, the alkylene oxide units are ethylene or propylene oxide units, namely - (CH₂CH₂O)ₓRₐₒ or -(CH₂CH₂CH₂O)ₓRₐₒ, wherein x and Rₐₒ are as described above. Most preferably, the alkylene oxide units are such ethylene oxide units.

The term "alkylene imine" refers to a radical derived from a saturated aliphatic chain containing alkylene imine units which is bound to rest of the molecule through a single bond. It may be linear or branched. The alkylene portion refers to an alkane-derived diradical. The alkylene oxide radical can be depicted through general formula -(alkylene-NH)ₓRₐₒ, wherein x is from 1 to 5, more preferably 1 or 2; the alkylene portion comprises from 1 to 16 ("C₁-C₁₆ alkylene"), preferably from 1 to 8 ("C₁-C₈ alkylene"), even more preferably from 1 to 4 ("C₁-C₄ alkylene") carbon atoms; and Rₐₒ is H or alkyl as described above, preferably H or methyl, more preferably H. Preferably, the alkylene imine units are ethylene or propylene imine units, namely - (CH₂CH₂NH)ₓRₐₒ or -(CH₂CH₂CH₂ NH)ₓRₐₒ, wherein x and Rₐₒ are as described above. Most preferably, the alkylene imine units are such ethylene imine units. The N-H group may also be substituted by an N-alkyl group, wherein alkyl has the meaning described above.

The term "halogen" refers to bromine, chlorine, iodine or fluorine.

The term "polyonium" refers to a polycation comprising two or more organic onium cations as described below which are preferably connected intramolecularly by organic linkers, such as those comprising 1 to 8 carbons, such as C₁-C₈ alkylene, e.g. methylene, ethylene, propylene or butylene. The molecular structure of polyonium cations used in the present invention can be, without being limited thereto, a linear-chain structure, branched structure, and ring-shaped structure. Examples of polyonium include polyammonium, polyphosphonium, polypyridinium, polypyrrolidonium, polyimidazolium, polyimidazolinium and polysulfonium cations. In an embodiment, the polyonium comprises at least 10 onium cations, such as at least 100 or at least 1000 onium cations. In an embodiment, the polyonium comprises up to 3000 onium cations. The terms fluorination reagent, chlorination reagent, bromination reagent and iodination reagent, refer to compounds that are capable to react with the compound of formula (IV) by releasing an electrophilic fluorine, a chlorine, a bromine or a iodine atom, respectively, thus forming a new S-F, S-Cl, S-Br or C-I bond while oxidizing the central S from oxidation state (IV) to oxidation state (VI). Examples of these reagents include 1-Chloromethyl-4-fluoro-1,4-diazoniabicyclo[2.2.2]octane bis(tetrafluoroborate) (also known as F-TEDA or selectfluor), N-fluorobenzenesulfonimides, such as N-Fluorobenzenesulfonimide (NFSI) and fluoropyridinium salts, such as N-fluoropyridinium triflatetetrafluoroborate; bromination reagents, such as N-Bromosuccinimide (NBS); chlorination reagents, such as N-Chlorosuccinimide (NCS); iodination reagents as N-Iodosuccinimide (NIS).

The term "alkylation reagent" refers to compounds that are capable to react with the compound of formula (IV) by releasing an electrophilic alkyl group (as defined above) optionally substituted with at least one F, an alkenyl, an aryl, -N(R₆)(R₇) or OR₈ (as also defined elsewhere herein), thus forming a new S-C bond while oxidizing the central S from oxidation state (IV) to oxidation state (VI). Examples of alkylation reagents are iodoalkanes (e.g. iodomethane), alkyl triflates (e.g. methyl triflate), trimethyloxonium tetrafluoroborate, alkyl bromides (e.g. CH₃Br, CH₂CH₃Br).

The term "fluoroalkylation reagent" refers to compounds that are capable to react with the compound of formula (IV) by releasing an electrophilic fluoroalkyl group, namely an alkyl group (as defined above) where at least one C-H group is substituted by a C-F groups, thus forming a new S-C bond. Preferably, a fluoroalkylation reagent is a perfluoroalkylation reagent able to release an electrophilic perfluoroalkyl group (R_{F}), where all C-H groups are substituted by C-F groups, thus forming a new S-R_{F} bond Typical examples of perfluoroalkylation reagents are Togni reagents (I and II), Langlois reagent (CF₃SO₂Na), Umemoto's reagent, which are either commercial or can be accessed by reported methods (for example, Chem. Commun., 2016, 52, 4049-4052).

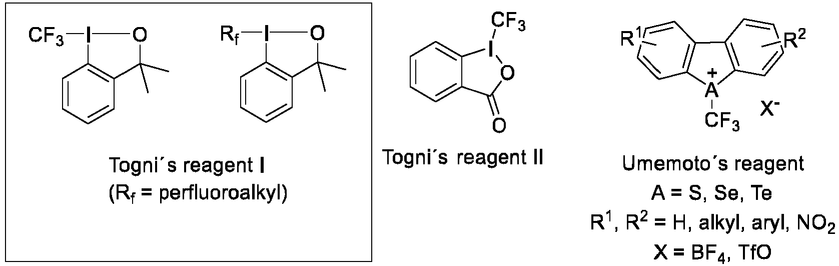

The term "amination reagent" refers to compounds that are capable to react with the compound of formula (IV) by releasing an electrophilic N(R₄)(R₅) group (R₄ and R₅ are as defined above), thus forming a new S-N(R₄)(R₅) bond. Typical examples of suitable aminations reagents are chloroamines ClN(R₄)(R₅), which can be either commercially available or prepared by treating the corresponding secondary amines HN(R₄)(R₅) with aqueous hypochlorous acid.

In an embodiment, when double substitution at an amine nitrogen with a given group, such as Y₂, R'₂, (R₄)(R₅) or (R₆)(R₇), is indicated, each of the two specific substituents chosen from that group is selected independently from the other. In another embodiment, said substituents are the same.

The inventors have found that a method for preparing the ionic compounds of formula I according to the procedure detailed in the description and examples of the present disclosure display several advantages.

In particular, the method of the invention can be carried in a one-pot manner. A "one-pot" reaction implies that a sequence of steps (in the present case, two steps) are performed consecutively in the same reaction vessel, meaning that further experimental operations such as solvent concentration or evaporation, extraction and/or other purification techniques are not required between a step and the subsequent one. This represents a considerable advantage in terms of reagents and time economy.

Another advantage of the present method is that the reaction conditions are milder than those in previous reports. For example, the method can be carried out at temperatures comprised between -20 °C and room temperature in contrast to temperatures of -50 °C that are required to obtain some of the ionic compounds described in previous reports (J. Chem. Soc., Perkin Trans. 1, 2002, 1887-1889).

Further, the method of the invention features high step-and reagent-economy as the longest linear sequence is carried out in 4 steps including the optional metal-cation exchange step while the traditional route (see background of the invention) is performed in a 5-steps linear sequence. Furthermore, TMSCF₃ (prepared from BrCF₃, which is environmentally harmful) is avoided. The excess of CsF (2 equivalents) is replaced by a nearly stoichiometric amount of reagents (such as F-TEDA in the example below). Finally, the intermediates RS(O)(NSO₂CF₃)F (R is CF₃ or Ph) in previous reports are poorly stable towards moisture and require a complex purification procedure while the current method is performed in a one-pot manner as said above, therefore exposure to moisture and air is minimized and the reagents and final products are stable and easy to handle.

Finally, the modulability with regards to the groups R¹, R² and E allows to obtain a wide scope of ionic compounds.

As detailed herein below, the method of the present invention is performed in a one-pot twostep sequence and, optionally, additional steps may be performed.

In the context of the present invention, the terms "ionic compound" and "salt" will be used interchangeably. The ionic compounds synthesized according to the process of the invention comprise a cation M and an anion of formula: 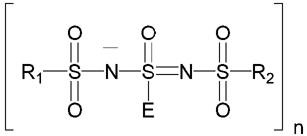 wherein the subscript "n" is a positive integer number that refers to the number of anions needed to neutralize the charge of the cation M. The negative charge in the anion is represented for the sake of illustration on one nitrogen atom, however, it is a delocalized negative charge that is distributed due to resonance over the two nitrogen atoms and the five oxygen atoms in the anion of the general formula **I.** Even though one N-S bond (where S is the central sulphur atom) is graphically represented with a single bond while the other N-S bond (again, where S is the central sulphur atom) with a double bond, it has to be understood that both N-S bonds have an intermediate character between that of a single and double bond due to resonance.

The cation M in the compound of formula I can be a number of organic, organometallic and inorganic species that are able to carry one or more positive charges.

In a preferred embodiment, M is an alkali metal, that is, a metal of the group 1 of the periodic table that forms a monovalent cation M⁺. In a preferred embodiment, M is an alkali metal chosen from Li⁺, Na⁺, K⁺ and Cs⁺. More preferred alkali metals are Li⁺, Na⁺ and K⁺. Even more preferably, the alkali metal is Li⁺.

In another embodiment, M is an alkaline earth metal, that is, a metal of the group 2 of the periodic table that forms a divalent metal cation M²⁺. In a preferred embodiment, the alkaline earth metal is selected from Mg²⁺, Ca²⁺ and Ba²⁺.

In another embodiment, M is a transition metal with a valency comprised between 1 and 3. In a preferred embodiment, M is a transition metal selected from the group consisting of Cu²⁺, Zn²⁺, Fe²⁺ and Al³⁺.

In another embodiment, M is a rare-earth metal.

In another embodiment, M is a onium or polyonium cation.

In a preferred embodiment, the cation M is selected from the group of metal cations consisting of K⁺, Li⁺, Na⁺, Cs⁺, Mg²⁺, Ca²⁺, Ba²⁺, Cu²⁺, Zn²⁺, Fe²⁺, or Al³⁺.

In another embodiment, M in the compound of formula I is:
- a monovalent or divalent cation, selected from ions of alkali metals, of alkaline earth metals, of transition metals and of rare-earth metals;
- an organic onium cation; or
- an organometallic cation.

In a preferred embodiment, the method of the invention affords an ionic compound of formula **I** wherein M is Li⁺, Na⁺ or K⁺. More preferably, M is K⁺ and thus m=1.

In another embodiment, the cation M is an organic onium cation, namely a cation obtained by the protonation of a mononuclear hydride of a pnictogen (group 15 of the periodic table), chalcogen (group 16), or halogen (group 17) atom; or a derivative thereof wherein at least one H radical is replaced with a C₁-C₈ alkyl, a C₆-C₁₀ aryl or a heteroaryl radical. Examples of group 15 onium cations are ammonium (NH₄⁺), phosphonium (PH₄⁺) and arsonium (AsH₄⁺). Thus, onium cations can also be considered as monovalent cations. Preferred organic onium cations are nitrogen-comprising onium cations such as pyrrolidinium, imidazolium, imidazolinium ions. The terms "C₁-C₈ alkyl" and "C₆-C₁₀ aryl" have the meaning described above. The term "heteroaryl" refers to an aromatic monocyclic or bicyclic system containing from 5 to 10 ring atoms containing one or more, specifically one, two or three ring heteroatoms independently selected from O, N and S, and the remaining ring atoms being carbon.

In another embodiment, the cation M is an organometallic cation. This can be monovalent, divalent, trivalent or polyvalent. The organometallic cation can be chosen from metalloceniums. For example, mention may be made of the cations derived from ferrocene (i.e. ferrocenium ion), from titanocene, from zirconocene, from an indocenium or from an arene metallocenium. It can also be chosen from metal cations coordinated by atoms such as O, S, Se, N, P or As, borne by organic molecules, in particular in the form of carbonyl, phosphine or porphyrine ligands optionally containing chirality. M can also be a metal cation bearing alkyl groups, such as those containing from 1 to 10 carbon atoms, for example a trialkylsilyl or dialkylstannyl derivative; in this case, M is linked to the anion [R₁-S(O)₂-N⁻-S(O)(R₂)-N-S(O)₂-R₃] via a very labile covalent bond and the compound behaves like a salt. The cation M can also be the cationic oxidized form of methylzinc, phenyl mercury, trialkyltin or trialkyllead (wherein alkyl means "C₁-C₈ alkyl" as defined above), chloro[ethylenebis(indenyl)]zirconium(IV) or tetrakis-(acetonitrile)palladium(II) cations.

In a particularly preferred embodiment, the cation M is a polyonium cation. A polyonium cation can be introduced by the cation exchange step after step b). The cation exchange step can be carried out via standard methods known in the art for cation exchange, for example by dissolving the compound I with a polyonium halide in the same solvent (e.g. water) and let them react until completion. In a preferred embodiment the polyonium cation is a polyammonium, polypyrrolidonium, polyimidazolium, polyimidazolinium cation, preferably a polyammonium, polyimidazolium, or polyimidazolinium cation.

In a preferred embodiment, the polypyrrolidonium cation has the following general formula: 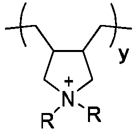 wherein each R is independently alkyl, alkenyl, aryl, or alkylene oxide, wherein these terms have the meaning described above; and wherein y denotes the number of repeating onium ion units comprised in the polyonium. Preferably, the alkyl and alkenyl are respectively a C₁-C₁₂ alkyl and a C₂-C₁₂ alkenyl. Preferably, the alkylene oxide is ethylene oxide or propylene oxide; preferably in the alkylene oxide n is 2, even more preferably n is 1. In a preferred embodiment, the polypyrrolidonium cation is polyDADMA, that is polydiallyldimethylammonium.

In another preferred embodiment, the polyimidazolium cation is one wherein the imidazolium cations do not form part of the polyimidazolium backbone, but are rather comprised in sidechains thereof.

In a particular embodiment, the polyimidazolium cation may have one of the following general formulas: 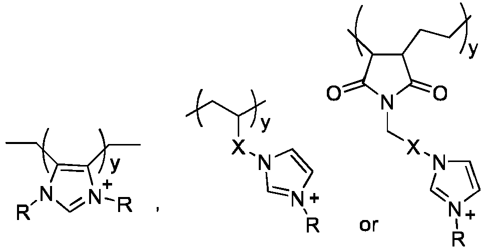 where each R is independently alkyl, alkenyl, aryl, or alkylene oxide, wherein these terms have the meaning described above; X is a spacer corresponding to an alkylene (preferably a C₁-C₆ alkylene), a phenylene (-C₆H₄- optionally with ortho, meta or para substitution on the aromatic ring) or a -C(=O)O- group; and y denotes the number of repeating onium ion units comprised in the polyonium. Preferably, the polyimidazolium cation is 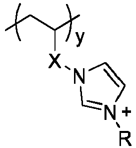 wherein R, X and y are defined as above.

In another preferred embodiment, the polyimidazolinium cation is selected from one of the following structures: 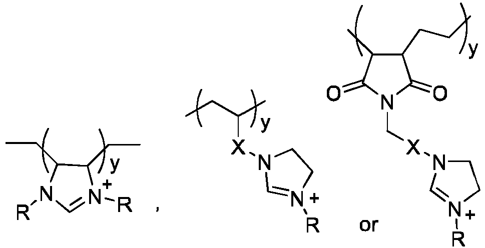 wherein each R, X and y are defined as above for polyimidazoliums.

The anion of the salt of formula (I) has two R fragments, R₁ and R₂, which are independently selected from -Y, -OY, -SY, -NY₂ and halogen.

In one embodiment, R₁ and R₂ are identical. In another embodiment, R₁ and R₂ differ from each other.

In one embodiment, both R₁ and R₂ are independently selected from -Y, -OY, -SY, -NY₂. In another embodiment, one of R₁ and R₂ is a halogen, preferably F, and the other is selected from -Y, -OY, -SY, -NY₂, preferably from -Y and -NY₂. In another embodiment, both R₁ and R₂ are halogen, preferably both are F.

In another embodiment, R₁ and R₂ are independently selected from -Y, -NY₂ and halogen.

In another embodiment, R₁ and R₂ are independently selected from -Y and -NY₂.

In another preferred embodiment, at least one of R₁ and R₂ is -Y. More preferably, both R₁ and R₂ are -Y.

In another preferred embodiment, at least one of R₁ and R₂ is -NY₂. More preferably, both R₁ and R₂ are -NY₂.

In a preferred embodiment, Y represents an organic radical selected from alkyl, alkenyl, alkynyl, aryl, alkylaryl, arylalkyl, alkylene oxide or alkylene imine, optionally substituted with at least a substituent selected from the group consisting of F, Cl, Br, I, -CN, -OR', -SR', -NR'₂, wherein each R' is independently selected from H, alkyl, alkylene oxide or alkylene imine.

In a more preferred embodiment, Y represents an organic radical selected from alkyl, aryl, alkylaryl, arylalkyl, alkylene oxide or alkylene imine, optionally substituted with at least a substituent selected from the group consisting of F, Cl, Br, I, -CN, -OR', -SR', -NR'₂, wherein each R' is independently selected from H, alkyl, alkylene oxide or alkylene imine.

In a preferred embodiment, the organic radical is substituted with at least one F, Cl, Br or I. The substitution can occur at any position of the aliphatic chain or aromatic ring present in the radical, meaning that at least one aliphatic or aromatic C-H group is substituted by F, Cl, Br or I. More preferably, Y represents an organic radical selected from fluorinated or perfluorinated alkyl, alkenyl, alkynyl, aryl, alkylaryl, arylalkyl, alkylene oxide or alkylene imine, i.e. any aliphatic or aromatic C-H present in the organic radical is partially or completely substituted with F atoms.

In one embodiment, at least one of the groups R₁ and R₂ is independently selected from -Y, - OY, -SY, -NY₂.

In a preferred embodiment, at least one of R₁ and R₂ is -Y. More preferably, Y represents an organic radical chosen from fluorinated or perfluorinated alkyl, alkenyl, alkynyl, aryl, alkylaryl, arylalkyl, alkylene oxide or alkylene imine, and more preferably Y represents an organic radical chosen from fluorinated or perfluorinated alkyl.

In another preferred embodiment, at least one of R₁ and R₂ is -NY₂. More preferably, Y represents an organic radical chosen from fluorinated or perfluorinated alkyl, alkenyl, alkynyl, aryl, alkylaryl, arylalkyl, alkylene oxide or alkylene imine, and more preferably Y represents an organic radical chosen from fluorinated or perfluorinated alkyl.

In a preferred embodiment, Y represents an organic radical as defined above. In a more preferred embodiment, the organic radical is optionally substituted with at least one F, Cl, Br or I. More preferably, the organic radical is optionally fluorinated or perfluorinated, i.e. the H atoms at any aliphatic chain or aromatic ring present in the radical are partially or completely substituted by F atoms. In an embodiment, the organic radical is fluorinated or perfluorinated. In a preferred embodiment, at least one of the groups R₁ and R₂ is F and the remainder, if any, is -Y, wherein Y is as described elsewhere herein. More preferably, at least one of the groups R₁ and R₂ is F and the remainder is selected from -Y, wherein Y represents an organic radical chosen from fluorinated or perfluorinated alkyl, alkenyl, alkynyl, aryl, alkylaryl, arylalkyl, alkylene oxide or alkylene imine, and more preferably Y represents an organic radical chosen from fluorinated or perfluorinated alkyl.

In another preferred embodiment, at least one of the groups R₁ and R₂ is F and the remainder, if any, is -NY₂, wherein Y is as described elsewhere herein. More preferably, at least one of the groups R₁ and R₂ is F and the remainder, if any, is -Y, wherein Y represents an organic radical chosen from fluorinated or perfluorinated alkyl, alkenyl, alkynyl, aryl, alkylaryl, arylalkyl, alkylene oxide or alkylene imine, and more preferably Y represents an organic radical chosen from fluorinated or perfluorinated alkyl.

In a preferred embodiment, at least one of the groups R₁ and R₂ is F and the remainder, if any, is independently selected from -OY, -SY, -NY₂, preferably from -OY and/or -NY₂, wherein Y is as described elsewhere herein. More preferably, Y is alkyl, alkenyl, alkynyl or alkylene oxide.

In a preferred embodiment, at least one of the groups R₁ and R₂ is F and the remainder, if any, is independently selected from -Y, -OY, -SY, -NY₂, wherein Y for -Y is selected from fluorinated or perfluorinated alkyl, alkenyl, alkynyl, aryl, alkylaryl, arylalkyl, alkylene oxide or alkylene imine, and more preferably selected from fluorinated or perfluorinated alkyl; and Y for -OY, -SY, -NY₂ is alkyl, alkenyl, alkynyl or alkylene oxide.

In a preferred embodiment:
- when Y is alkyl, then it is fluorinated or perfluorinated; or particularly
- when Y is alkyl, alkenyl, alkynyl, then it is fluorinated or perfluorinated; or more particularly
- when Y is alkyl, alkenyl, alkynyl, aryl, alkylaryl, arylalkyl, alkylene oxide or alkylene imine, then it is fluorinated or perfluorinated.

In a preferred embodiment:
- when Y in any -Y group is alkyl, then it is fluorinated or perfluorinated; or particularly
- when Y in any -Y group is alkyl, alkenyl, alkynyl, then it is fluorinated or perfluorinated; or more particularly;
- when Y in any -Y group is alkyl, alkenyl, alkynyl, aryl, alkylaryl, arylalkyl, alkylene oxide or alkylene imine, then it is fluorinated or perfluorinated.

In an embodiment, in the ionic compound of formula I as defined above, Y groups are optionally substituted. In another embodiment, they are substituted. Substitution is preferably at any aliphatic or aromatic C-H bond present in the organic radical.

In a specific embodiment, each Y independently represents an alkyl radical as described above which is fluorinated or perfluorinated, more preferably an alkyl radical comprising from 1 to 4 carbon atoms which is fluorinated or perfluorinated, even more preferably Y represents CF₃. In a more specific embodiment, at least one of R₁ and R₂ is F and Y at any -Y group at the remainder of R₁ and R₂ is selected from an alkyl radical which is fluorinated or perfluorinated, more preferably an alkyl radical comprising from 1 to 4 carbon atoms which is fluorinated or perfluorinated, even more preferably Y represents CF₃.

In a more specific embodiment, at least one of R₁ and R₂ is F and the remainder of R₁ and R₂, if any, is selected from -Y, wherein Y is an alkyl radical which is fluorinated or perfluorinated, more preferably an alkyl radical comprising from 1 to 4 carbon atoms which is fluorinated or perfluorinated, even more preferably Y represents CF₃.

Examples of perfluorinated radicals Y are -CF₃, -CF₂CF₃, -CF₂(CF₂)ₙCF₃ (n = 1 to 7), - CF(CF₃)₂, -CF₂CF(CF₃)₂, -CF(CF₃)(CF₂)ₙCF₃ (n = 1 to 5), -CF₂CF(CF₃)(CF₂)ₙCF₃ (n = 1 to 4).-CF=CF₂, -CF=CFCF₃, -CF₂CF=CF₂, -CF=C(CF₃)₂, -CF=CFCF₂CF₃, -CF₂CF=CF₂CF₃, - CF₂CF₂CF=CF₂, -C≡CCF₃, -CF₂C≡CF, -C≡CCF₂CF₃, -CF₂CF₂C≡CF, -C(O)CF₃, - C(O)(CF₂)ₙCF₃ (n = 1 to 6), -C(O)C₆F₅, -C(O)C₆F₄CF₃, -C₆F₅, -C₆F₄CF₃, -C₆F₄CF₂CF₃, - C₆F₃(CF₃)CF₃ (all ortho, meta and para isomers), -CF₂C₆F₅, -CF₂CF₂C₆F₅, -CF₂C₆F₄CF₂ (all ortho, meta and para isomers).

In another preferred embodiment, at least one of the groups R₁ and R₂ is F and the remainder, if any, is a group -Y, wherein Y is a polymeric group comprising repeating units selected from alkylene oxide, alkylene imine, styrene, acrylate, maleimide, phosphazene, siloxane, vinyl alcohol, vinyl amine or mixtures thereof, preferably the polymeric group comprises repeating units selected from alkylene oxide, alkylene imine, acrylate, maleimide, phosphazene, siloxane, vinyl alcohol, vinyl amine or mixtures thereof, more preferably the polymeric group comprises repeating units selected from alkylene oxide, acrylate or maleimide repeating units. These repeating units have the same meaning and particular embodiments as provided for the conductive polymer below. In another preferred embodiment, Y is a polymeric group comprising repeating units of alkylene oxide or alkylene imine, more preferably of alkylene oxide. The term "acrylate" has to be understood in a broad sense as including derivatives such as alkylacrylates and alkyl esters thereof, preferably methylmethacrylate.

In a preferred embodiment, the alkylene oxide is ethylene oxide, propylene oxide or a copolymer of these two, wherein the copolymer preferably has the following structure: 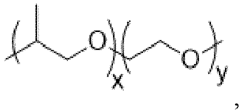 wherein x and y subscripts are positive integers corresponding to a number of repeating units in the polymeric chain and the sum of x and y preferably amounts to at least 100 or at least 1000, and preferably up to 115.000.

Examples of repeating units or monomers are shown in parentheses below (the bond outside the parentheses does not define a methyl group but denotes a bond to the rest of the polymer, such as to other repeating units, to terminal groups, to the rest of the compound of formula I, or to the linker): 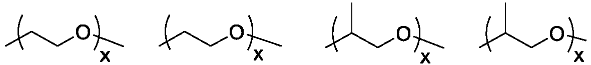 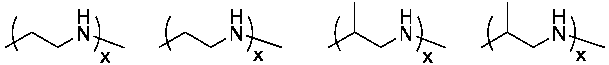 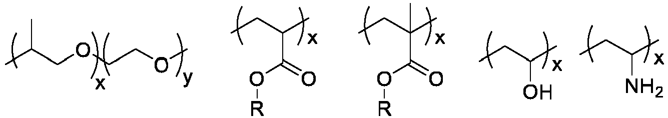 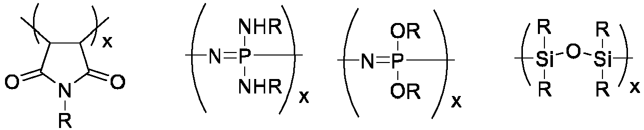 wherein R is alkyl as defined elsewhere throughout the text and x and y subscripts are positive integers corresponding to a number of repeating units in the polymeric chain and is preferably at least 100 or at least 1000, and preferably up to 115.000. The repeating unit of the polymeric group may be connected to the sulfur atoms of the compounds of formula via carbon atom or via a heteroatom (O, N, P or Si). The polymeric groups could be considered as a particular case of Y being a substituted aliphatic chain bearing alkylene oxide, alkylene imine, acrylate, maleimide, vinyl alcohol, vinyl amine repeating units. These polymeric groups could be obtained from the corresponding sulfonamide originally bearing the repeating unit or from a sulfonamide that can be later modified to introduce the repeating unit. For example, a sulfonamide with the formula NH₂-SO₂(CH₂)ₙ-OY' or NH₂-SO₂-(CH₂)ₛ-NY'₂ where Y' is a protecting group, can be later substituted by an alternative Y, which is a polymeric group. (CH₂)ₙ in the previous formula aims to indicate that OY' and NY'₂ can be directly linked to the sulfur (n = 0) or there might be a linker in between (n = 1, 2, 3, etc.). More details were provided above for alkylene and alkylene imine substitution. Another strategy includes the substitution of a sulphonamide bearing a S-halogen bond with an alternative Y, which is a polymeric group. Such strategies are well documented in literature, for example Zhang et al., Chem. Soc. Rev., 2017, Feb 6;46(3):797-815) describes the following two strategies to form conductive polymers (paragraph 2):
a) polymerization of a salt polymer (such as a lithium salt) containing a certain monomer; and;
b) selective modification of an existing polymer.

Terminal group(s) comprised in the polymeric group depend on the type of repeating unit that terminates the polymer chain and the type of reaction employed for polymerization. In an embodiment, the terminal group(s) are H or alkyl as described above, preferably H or methyl, more preferably methyl.

In a particular embodiment, the polymer group has a molecular weight of between 50 Dalton and 5000000 Dalton (preferably measured by gel-permeation chromatography).

The anion of the salt of formula (I) has also a E fragment attached to the central S atom, which is selected from F; Br; Cl; I; -N(R₄)(R₅), wherein R₄ and R₅ are independently selected from H, an alkyl and an ethylene oxide chain having from 1 to 5 repeating units of ethylene oxide; an alkyl optionally substituted with at least one F, -N(R₆)(R₇) or OR₈, wherein R₆, R₇ and R₈ are independently selected from H and alkyl; and an arylalkenyl. Preferably, the ethylene oxide chain has from 1 to 3 repeating units of ethylene oxide.

In another preferred embodiment, E is selected from F; Br; Cl; I; -N(R₄)(R₅), wherein R₄ and R₅ are independently selected from H, an alkyl and an ethylene oxide chain having from 1 to 5 repeating units of ethylene oxide; and an alkyl optionally substituted with at least one F, N(R₆)(R₇) or OR₈, wherein R₆, R₇ and R₈ are independently selected from H and alkyl.

In another preferred embodiment, E is selected from F; an alkyl optionally substituted with at least one F; and N(R₄)(R₅), wherein R₄ and R₅ are independently selected from H, an alkyl and an ethylene oxide chain having from 1 to 5 repeating units of ethylene oxide.

More preferably, E is selected from F, an alkyl substituted with at least one F, and N(R₄)(R₅), wherein R₄ and R₅ are independently selected from an ethylene oxide chain having from 1 to 3 repeating units of ethylene oxide. Even more preferable, E is F or CF₃, and much more preferable E is F.

In a preferred embodiment, the method of the invention affords an ionic compound of formula **I,** wherein E is F or CF₃, and the groups R¹ and R² are independently selected from -Y, -OY and NY₂, preferably from -Y, wherein Y is as described elsewhere herein. Preferably, Y is a partially fluorinated or perfluorinated alkyl, alkenyl, alkynyl, aryl, alkylaryl, arylalkyl.

In a preferred embodiment, the method of the invention affords an ionic compound of formula **I,** wherein E is F or CF₃, and the groups R¹ and R² are independently selected from -Y and - OY, preferably from -OY, wherein Y is as described elsewhere herein. More preferably, Y is alkyl, alkenyl, alkynyl or alkylene oxide.

In a preferred embodiment, the method of the invention affords an ionic compound of formula **I,** wherein E is F or CF₃, and the groups R¹ and R² are independently selected from -Y and NY₂, preferably from -NY₂, wherein Y is as described elsewhere herein. More preferably, Y is alkyl, alkenyl, alkynyl or alkylene oxide.

In another preferred embodiment, the method of the invention affords an ionic compound of formula **I,** wherein E is F or CF₃, and at least one of R¹ and R² is halogen, preferably F, and the remaining is selected from -Y and -OY, preferably from -Y, wherein Y is as described elsewhere herein. In another preferred embodiment, E is F and R¹ and R² are a halogen, preferably F.

In another embodiment, the method of the invention affords an ionic compound of formula **I,** wherein M is Li⁺, Na⁺ or K⁺;
E is F and the groups R¹ and R² are independently selected from -Y and -OY, preferably from -Y, wherein Y represents an organic radical, selected from alkyl, alkenyl, alkynyl, aryl, alkylaryl, arylalkyl, alkylene oxide or alkylene imine, optionally substituted with at least one F, Cl, Br or I, more preferably any aliphatic or aromatic C-H present in the radical is partially fluorinated or perfluorinated.

In a most preferred embodiment, the method of the invention affords the ionic compound of formula **I,** wherein E is F and:
- when Y is alkyl, then it is partially fluorinated or perfluorinated; or particularly
- when Y is alkyl, alkenyl, alkynyl, then it is partially fluorinated or perfluorinated; or more particularly
- when Y is alkyl, alkenyl, alkynyl, aryl, alkylaryl, arylalkyl, alkylene oxide or alkylene imine, then it is partially fluorinated or perfluorinated.

In another embodiment, R¹ and R² are the same.

In a preferred embodiment:
- when Y in any -Y group is alkyl, then it is fluorinated or perfluorinated; or particularly
- when Y in any -Y group is alkyl, alkenyl, alkynyl, then it is fluorinated or perfluorinated; or more particularly;
- when Y in any -Y group is alkyl, alkenyl, alkynyl, aryl, alkylaryl, arylalkyl, alkylene oxide or alkylene imine, then it is fluorinated or perfluorinated.

In another embodiment, R¹ and R² are the same and Y is an alkyl radical comprising from 1 to 4 carbon atoms which is partially fluorinated or perfluorinated, preferably the alkyl radical is perfluorinated methyl (CF₃).

In another embodiment, R¹ is different from R².

### Step a)

Step a) of the process of the invention comprises the reaction of a *N*-(sulfinyl)sulfonamide of formula (II) R¹SO₂N=S=O with a sulphonamide salt of formula (III) R²SO₂N(M)ₘ, wherein
- R¹ and R² are as defined elsewhere herein,
- M is a proton, a monovalent cation, a divalent cation selected from ions of alkali metals, of alkaline earth metals, of transition metals, of rare-earth metals; an organic onium or polyonium cation; or an organometallic cation;
- m is 1 if M is a divalent cation or 2 if M is a proton or a monovalent cation.

When M is a proton, the reaction can still take place however it may require the presence of a base. Suitable bases for carrying out the reaction between compounds of formula (II) and (III) (when M is proton) are hydroxide and hydrides of alkali and alkaline earth metals, non-nucleophilic bases, as tertiary amines, amides of alkali and alkaline earth metals or organometallic compounds; preferably suitable bases are non-nucleophilic bases, as tertiary amines, amides of alkali and alkaline earth metals or organometallic compounds.

The reaction between a compound of formula (II) and a compound of formula (III) generates a dianionic compound of formula (IV) [R¹SO₂-N⁻-S(=O)-N⁻-SO₂R²][M]ₘas defined above.

Compounds of formula (II) R¹SO₂N=S=O can be obtained by reaction of the corresponding sulphonamide R¹SO₂NH₂ with SOCl₂ (for example, J. Chem. Soc., Perkin Trams. 1, 2002, 1887-1889 and references therein).

Sulphonamide salts of formula (III) R²SO₂N(M)ₘ can be obtained by reaction of the corresponding R²SO₂NH₂ with a suitable base able to extract the protons of the amino group in an aprotic solvent. Examples of suitable bases include an alkali or alkaline earth metal hydroxide, alkoxide, carbonate, hydride, amide, such as sodium hydroxide, potassium hydroxide, sodium methoxide, sodium ethoxide, potassium tert-butoxide, sodium carbonate, potassium carbonate, cesium carbonate or potassium hydride, while examples of suitable aprotic solvents include THF, diethyl ether, methyl tert butyl ether (MTBE), 1,4-dioxane, MeTHF, acetonitrile, dichloromethane, ethyl acetate nitrobenzene, 1,2-dichloroethane or mixtures thereof. The same compound R²SO₂NH₂ in presence of a base can be used in step a) to react with R¹SO₂N=S=O. The cation M in both the compound of formula (III) and dianionic sulfonate of formula (IV) can be a number of organic, organometallic and inorganic species that are able to carry one or two positive charges.

Starting sulfonamides R¹SO₂NH₂ and R²SO₂NH₂ (such as alkyl, aryl and alkylaryl sulphonamides) or sulfonyl chlorides are widely commercially available from different chemical vendors such as Sigma-Aldrich. Alternatively, sulphonamides R¹SO₂NH₂ and R²SO₂NH₂ can be obtained by the treatment of sulfonyl chloride precursors, i.e., R¹SO₂Cl and R²SO₂Cl, with ammonia, either gas or aqueous solution. Furthermore, sultones and sultams can be used as sulfonyl chloride surrogates to include alkylene oxide and alkylene imine in different ways (see Chapter 19). Strategies to access alkylene oxide/alkylene imine sulfonamides include a) using a sultone/sultam already containing the alkylene oxide/alkylene imine moiety or in a more general way ether/amine moieties in the ring (see Chapter 19, page 855), b) via simple hydrolysis/ammonolysis of sultones/sultams (see Chapter 19, pages 819 and 862), or c) via addition of aliphatic nucleophiles bearing the desired alkylene oxide/alkylene imine moiety (see Chapter 19, pages 835-836). Compounds like 1,3-propane sultone and propane sultam are commercially available reagents. Alkenyl and alkynyl derivatives can be prepared from corresponding sulfonyl chloride precursors (a) via ammonolysis of commercial alkenyl/alkynyl sulfonyl chlorides; b) via addition of Grignard reagents to sulfuryl chloride, see WO2010038465 A1 2010-04. In the particular case of R¹ and/or R² equal to OY, SY and NY₂ and Y = H, the alcohol, thiol or amine can be protected prior to avoid any undesired reactions of the sulfinyl sulfonamide, and then deprotected after obtaining compound of Formula I. An easy approach to get the sulfonamides would be to react sulfamoyl chloride with the corresponding protected alcohol, thiol or amine and subsequently deprotection of the alcohol, thiol or amine would follow according to general protection/deprotection strategies (Protecting Groups, Philip Kocienski, ISBN: 3-13-137003-3, Georg Thieme Verlag Stuttgart or Protective Groups in Organic Synthesis, Theodora W. Greene, Peter G. M. Wuts, 4th Edition, 2006, ISBN: 978-0-471-69754-1, Wiley).

In a preferred embodiment, M is an alkali metal, that is, a metal of the group 1 of the periodic table that forms a monovalent cation M⁺. In a preferred embodiment, M is an alkali metal selected from Li⁺, Na⁺, K⁺ and Cs⁺. More preferred alkali metals are Li⁺, Na⁺ and K⁺. Even more preferably, the alkali metal is K⁺. When M is an alkali metal, subscript m is equal to 2.

In another embodiment, M is an alkaline earth metal, that is, a metal of the group 2 of the periodic table that forms a divalent metal cation M²⁺. In a preferred embodiment, the alkaline earth metal is selected from Mg²⁺, Ca²⁺ and Ba²⁺. When M is an alkaline earth metal, subscript m is equal to 1.

In another embodiment, M is a transition metal with a valency of 1 or 2. In a preferred embodiment, M is a transition metal selected from the group consisting of Cu²⁺, Zn²⁺, Fe²⁺ and subscript m is equal to 1.

In a preferred embodiment, the cation M is selected from the group of metal cations consisting of K⁺, Li⁺, Na⁺, Cs⁺, Mg²⁺, Ca²⁺, Ba²⁺, Cu²⁺, Zn²⁺, Fe²⁺preferably the cation is K⁺.

In another embodiment, the cation M is an organic onium cation as defined above.

In another embodiment, the cation M is an organometallic cation as defined elsewhere herein.

In another particular embodiment, the R fragments, R₁ and R₂ are as defined elsewhere herein.

In a particular embodiment, the reaction between the N-(sulfinyl)sulfonamide of formula (II) and the sulphonamide salt of(III) is performed by adding R¹SO₂N=S=O dissolved in a suitable organic solvent to a suspension of R²SO₂N(M)ₘ suspended in the same or in a different organic solvent. In a preferred embodiment, the organic solvent is the same. Suitable solvents, both for dissolution of compound (II) and for a suspension of compound (III), are aprotic polar solvents such as THF, diethyl ether, methyl tert butyl ether (MTBE), dioxane, MeTHF, acetonitrile, dichloromethane, ethyl acetate or a combination thereof. Preferably, the solvent is a combination of THF with any of the aprotic polar solvents above. Even more preferably the solvent is THF.

The resulting mixture is let to react until complete conversion, typically for 20 minutes to 10 hours, more preferably for 1 hour.

The obtained compound is a dianionic compound of formula (IV) wherein the central sulfur atom has an oxidation state of (IV), thus rendering said intermediate compound a versatile synthon for oxidation reactions. Indeed, the dianionic compound of formula (IV) can react with electrophilic species to afford the corresponding sulfonate compounds of formula (I) where the central sulfur atom is in its highest oxidation state, S(VI).

Compound of formula (IV) can be purified, isolated and then used for the subsequent steps.

However, in a preferred embodiment of the present invention, step a) and step b) are performed in a one-pot manner. In this case, a purification step of the compound of formula (IV) is avoided, thus rendering the overall process less time-consuming and wasteful.

### Step b

As said earlier, the compound of formula (IV) [R¹SO₂-N⁻-S(=O)-N⁻-SO₂R²][M]ₘ obtained following step a) as described above, is susceptible of reaction with an appropriate electrophilic precursor at the central sulfur atom.

In an embodiment, the compound of formula (IV) is reacted with a reagent source of a E group as defined above. A reagent source of a E group can be understood as any neutral or charged reagent that may release an electrophilic group E able to accept an electron pair (from a nucleophile or electron-rich atom) to form a new bond. Such reagents suit the electron-rich character of the central sulfur atom in the compound of formula (IV) readily leading to the formation of a new E-S bond where the sulfur atom is in a VI oxidation state.

Typical reagents suitable to react with compound IV are fluorination reagents, chlorination reagents, bromination reagents, iodination reagents, alkylation reagents including fluoroalkylation reagents, and amination reagents as defined earlier in the text.

In a preferred embodiment, the reagent source of the E group is an reagent selected from F-TEDA, NFSI, N-fluoropyridinium tetrafluoroborate, NBS, NCS, NIS, ClN(R₄)(R₅) (wherein R₄ and R₅ are independently selected from H, an alkyl and an ethylene oxide chain having from 1 to 5 repeating units of ethylene oxide) or a trifluoromethylation reagent selected from Togni reagents (I and II), Langlois reagent (CF₃SO₂Na) and Umemoto's reagent. More preferably is F-TEDA.

In an embodiment, the reagent source of an E group is added neat.

In another embodiment, the reagent source of a E group is first dissolved in an organic solvent or in a mixture of organic solvents and then added to the solution of compound of formula (IV) resulting from step a) as described above.

In another embodiment, an organic solvent or a mixture of organic solvents is first added to the solution of compound of formula (IV), then the reagent source of a E group is added.

In a preferred embodiment, a mixture of THF and HFIP (hexafluoro-2-propanol) is first added to the solution of compound of formula (IV), then the reagent source of a E group is added. In a more preferred embodiment the ratio THF:HFIP is comprised between 10:1 to 1:10.

In another preferred embodiment, the reaction is let to react for 30 min to 48 hours until completion, preferably for 1 to 6 hours.

After conducting step b), the reaction provides an ionic compound of formula (I) as defined elsewhere above.

In an embodiment, the obtained ionic compound of formula (I) is subjected to purification. This step ensures the separation of said ionic compound from possible by-products in the reaction medium. In an embodiment, the purification of the ionic compound of formula (I) is performed by firstly evaporating the solvent and then extracting with aqueous acid and organic solvent.

Solvent evaporation can be performed by several techniques known to the person skilled in the art, for example by heating up the solution to a suitable temperature, applying reduced pressure or by a combination of heating and reduced pressure. A skilled person would be able to find the optimal pressure and temperature values for the evaporation of the solvent. In another embodiment, the extraction with aqueous acid and organic solvent is performed at least one time, at least two times or at least three times.

In another embodiment, the collected organic fractions from the at least one, at least two or at least three extractions is removed under reduced pressure.

The method of the present invention optionally comprise an additional step which allows functional groups interconversion to access other desired ionic compounds of formula (I) starting from the route as described herein above.

This is particularly the case wherein fragment E of the ionic compound of formula (I), obtained after conducting step b), is a fluorine atom.

Thus, in one embodiment, when E is a fluorine atom in the formula (I) obtained in step b), the process of the invention may further comprises a step wherein said fluorine atom is replaced by other nucleophile by a nucleophilic substitution reaction as taught in the textbook "The Chemistry of Sulfonic Acids, Esters, and Their Derivatives" (chapters 11, 21 and 22). More particularly, said fluorine atom is replaced by an alkyl radical or by a radical N(R₄)(R₅), wherein R₄ and R₅ are as defined above, by reacting said salt of formula (I) with an alkylation reagent or an amination reagent. The starting fluoride (E = F) would be equivalent to a sulfonyl halide, to a halosulfonic acid derivative, or to a sulphamic acid derivative. The reactivity of these compounds is well known and described in textbooks, such as "The Chemistry of Sulfonic Acids, Esters, and Their Derivatives" (DOI:10.1002/0470034394, Chapters 11, 21 and 22). Electron-withdrawing groups attached to the sulfur (VI) atom bearing the E group would make such E group more susceptible to nucleophilic attack, so it would be possible to substitute a fluoride, a chloride, a bromide or a iodine by another nucleophile. Nucleophilic hydrocarbons (e.g. TMS-alkanes) reacting with halosulfonic acid derivatives to afford sulphamic acid derivatives belong to common general knowledge. Additionally, O-, S- and N-containing compounds can also act as nucleophiles and a skilled person would routinely use them to replace a halide and obtain O-, S- and N-substituted products.

Thus, said reagents should be able to withdraw fluorine atoms from the E position and replace them with alkyl. In a particular embodiment, said reagents are organolithium and organomagnesium compounds, such as methyl lithium, phenyl magnesium bromide; or amino groups, such as ethyl-methyl amine, (methoxy methyl).(ethyl) amine. Such substitution should take place in basic conditions, in the presence of a base, such as secondary or tertiary amine, and at temperatures from 20 to 100 °C from 1 h to 48 h.

### Step c)

The ionic compound of formula (I) to be obtained according to the method of the invention contemplates the possibility of M being a trivalent cation or a polyonium cation. However, in the compound of formula (I) obtained after step (b), M is a monovalent cation or a divalent cation, so that an additional step is necessary to replace the M resulting from step (b) by a trivalent cation or polyonium cation.

Therefore, for this particular embodiment, the process of the invention further comprises a cation exchange reaction from the salt of formula (I) resulting from step b) to replace the monovalent or divalent cation M by a trivalent cation or a polyonium cation. Said monovalent or divalent cation is selected from ions of alkali metals, of alkaline earth metals, of transition metals, of rare-earth metals, an organic onium or a monovalent or divalent organometallic cation.

This cation exchange step can be carried out by widely known methods resulting in a compound of formula (I) where M is a trivalent cation or a polyonium cation.

In an embodiment, the cation exchange reaction is performed by dissolving the compound of formula (I) obtained after conducting step b) in a solvent, such as water, and then reacting it with a suitable salt having a trivalent cation. In a preferred embodiment, the salt is an aluminium salt, more preferably is Al₂(SO₄)₃.

In an embodiment, the cation exchange reaction is performed by dissolving the compound of formula (I) obtained after conducting step b) in a solvent, and then reacting it with a suitable metal, such as aluminum. In a preferred embodiment, the metal is aluminium foil.

In a preferred embodiment, the trivalent cation is selected from Al³⁺, Fe³⁺, Re³⁺, Sc³⁺, Cr³⁺, Mn³⁺, preferably is Al³⁺.

In another embodiment of the process of the invention, and irrespective of the cation M of the ionic compound of formula (I) obtained after conducting step b), said ionic compound of formula (I) can also be optionally subjected to a cation exchange step. In this embodiment, the ionic compound or salt of formula (I) is converted to another desired salt of formula (I') [R¹SO₂-N⁻-S(=O)(E)=N-SO₂R²]ₙ[M'], wherein M' is different from M, via standard methods known in the art for cation exchange. When M is replaced by M' which is a different monovalent or divalent cation, such cation is selected from the lists of monovalent and divalent cations already mentioned above.

In a preferred embodiment, M' is an alkali metal, more preferably is Li⁺. In an embodiment, in the cation exchange reaction M is K⁺ and is exchanged by M' being Li⁺.

In another embodiment, M' is a polyonium cation as defined elsewhere herein.

It would be evident to a skilled person that the charge of the polyonium is compensated by a sufficient number of anions in the resulting ionic compound of formula (I') (for example, if the polyonium has a charge of +10, then the subscript n in formula I will be 10 and the resulting ionic compound will be overall neutral).

In a preferred embodiment, the cation exchange reaction is performed to obtain an ionic compound of formula I', wherein M' is Li⁺, Na⁺ or a polyonium cation. In an embodiment, M' is Li⁺. In another embodiment, M' is a polyonium cation.

The cation exchange step can be carried out by widely known methods such as that described for example in Heng Zhang et al., Angew. Chem. Int. Ed., 2019, 131(23), 7829-7834).

In a particular embodiment, the cation exchange reaction is performed by dissolving the compound of formula (I) in a suitable solvent and then reacting it with a suitable salt to afford a salt of formula [R¹SO₂-N⁻-S(=O)(E)=N-SO₂R²][M'].

In a particular embodiment, the cation exchange reaction is performed by dissolving the compound of formula (I), wherein M is not Li⁺, in water and then reacting it with a suitable lithium salt to afford a salt of formula [R¹SO₂-N⁻-S(=O)(E)=N-SO₂R²][Li⁺]. In a preferred embodiment, the lithium salt is lithium carbonate.

In another embodiment, the cation exchange reaction is performed by dissolving the compound of formula (I) in water and then reacting it with a polyonium salt. In a preferred embodiment, the polyonium salt is a polyonium halide.

In a preferred embodiment, the cation exchange reaction takes place at a temperature between 0°C and 40 °C, preferably at a temperature between 20°C and 30 °C, more preferably at room temperature. In an embodiment, the reaction mixture is stirred for a sufficient time for the cation exchange to take place, preferably at least 5 min, at least 30 min, at least 1 hour, at least 2 hours, at least 4 hours, at least 8 hours, at least 16 hours and no longer than 24 hours. In another embodiment, the water solvent is subsequently removed under vacuum.

In another embodiment, the salt [R¹SO₂-N⁻-S(=O)(E)=N-SO₂R²]ₙ[M'] obtained after cation exchange is recrystallized. In a preferred embodiment, said salt is [R¹SO₂-N⁻-S(=O)(E)=N-SO₂R²][Li⁺] and is recrystallized from acetonitrile.

### Dianionic compound [R¹SO₂-N⁻-S(=O)-N⁻-SO₂R²][M]ₘ

The dianionic compound intermediate of formula (IV): 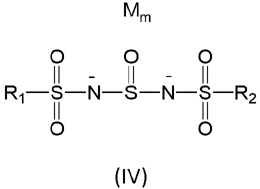 as obtained from step a) and as defined above constitutes another aspect of the present invention.

In a preferred embodiment, M is an alkali metal, that is, a metal of the group 1 of the periodic table that forms a monovalent cation M⁺. In a preferred embodiment, M is an alkali metal chosen from Li⁺, Na⁺, K⁺ and Cs⁺. More preferred alkali metals are Li⁺, Na⁺ and K⁺. Even more preferably, the alkali metal is Li⁺.

In another embodiment, M is an alkaline earth metal, that is, a metal of the group 2 of the periodic table that forms a divalent metal cation M²⁺. In a preferred embodiment, the alkaline earth metal is selected from Mg²⁺, Ca²⁺ and Ba²⁺.

In another embodiment, M is a transition metal selected from the group consisting of Cu²⁺, Zn²⁺ and Fe²⁺.

In a preferred embodiment, the cation M is selected from the group of metal cations consisting of K⁺, Li⁺, Na⁺, Cs⁺, Mg²⁺, Ca²⁺, Ba²⁺, Cu²⁺, Zn²⁺ and Fe²⁺.

In a particular embodiment, the dianionic compound intermediate of formula (IV) is characterized by M being a metal cation selected from K⁺, Li⁺, Na⁺, Cs⁺, preferably is K⁺.

In a particular embodiment, the dianionic compound intermediate of formula (IV) is characterized in that R¹ and R² are independently selected from:
- halogen,
- -Y, wherein Y represents:
   - an organic radical chosen from alkyl, alkenyl, alkynyl, aryl, alkylaryl, arylalkyl, alkylene oxide or alkylene imine, optionally substituted with at least a substituent selected from the group consisting of F, Cl, Br, I, -CN, -OR', -SR', -NR'₂, wherein each R' is independently selected from H, alkyl, alkylene oxide, alkylene imine; or
   - a polymeric group comprising repeating units selected from alkylene oxide, alkylene imine, acrylate, maleimide, phosphazene, siloxane, vinyl alcohol, vinyl amine or mixtures thereof; and
- -OY, -SY, -NY₂, wherein Y represents:
   - H or an organic radical selected from alkyl, alkenyl, alkynyl, aryl, alkylaryl, arylalkyl, alkylene oxide or alkylene imine, optionally substituted with at least a substituent selected from the group consisting of F, Cl, Br, I, -CN, -OR', -SR', -NR'₂, wherein R' is H, alkyl, alkylene oxide or alkylene imine; or
   - a polymeric group comprising repeating units selected from alkylene oxide, alkylene imine, acrylate, maleimide, phosphazene, siloxane, vinyl alcohol, vinyl amine or mixtures thereof;
wherein each of the two specific substituents Y at NY₂ and R' at NR'₂ is selected independently from the other.

In one embodiment, both R₁ and R₂ are independently selected from -Y, -OY, -SY, -NY₂. In another embodiment, one of R₁ and R₂ is a halogen, preferably F, and the other is selected from -Y, -OY, -SY, -NY₂. In another embodiment, both R₁ and R₂ are halogen, preferably both are F.

In another embodiment, R₁ and R₂ are independently selected from -Y, -NY₂ and halogen.

In another embodiment, R₁ and R₂ are independently selected from -Y and -NY₂.

In another preferred embodiment, at least one of R₁ and R₂ is -Y. More preferably, both R₁ and R₂ are -Y.

In another preferred embodiment, at least one of R₁ and R₂ is -NY₂. More preferably, both R₁ and R₂ are -NY₂.

In a preferred embodiment, Y represents an organic radical as defined above. In a more preferred embodiment, the organic radical is optionally substituted with at least one F, Cl, Br or I. More preferably, the organic radical is optionally fluorinated or perfluorinated, i.e. the H atoms at any aliphatic chain or aromatic ring present in the radical are partially or completely substituted by F atoms. In an embodiment, the organic radical is fluorinated or perfluorinated.

In a preferred embodiment, in the compound of formula (IV) at least one of the groups R₁ and R₂ is F and the remainder, if any, is -Y, wherein Y is as described elsewhere herein. More preferably, at one of the groups R₁ and R₂ is F and the other is -Y, wherein Y represents an organic radical selected from fluorinated or perfluorinated alkyl, alkenyl, alkynyl, aryl, alkylaryl, arylalkyl, alkylene oxide or alkylene imine, and more preferably Y represents an organic radical chosen from fluorinated or perfluorinated alkyl.

In another preferred embodiment, in the compound of formula (IV) at least one of the groups R₁ and R₂ is F and the remainder, if any, is -NY₂, wherein Y is as described elsewhere herein. More preferably, at least one of the groups R₁ and R₂ is F and the remainder, if any, is -Y, wherein Y represents an organic radical chosen from fluorinated or perfluorinated alkyl, alkenyl, alkynyl, aryl, alkylaryl, arylalkyl, alkylene oxide or alkylene imine, and more preferably Y represents an organic radical chosen from fluorinated or perfluorinated alkyl.

In a preferred embodiment, in the compound of formula (IV) at least one of the groups R₁ and R₂ is F and the remainder, if any, is selected from -OY, -SY, -NY₂, preferably from -OY and/or -NY₂, wherein Y is as described elsewhere herein. More preferably, Y is alkyl, alkenyl, alkynyl or alkylene oxide.

In a preferred embodiment, in the compound of formula (IV) at least one of the groups R₁ and R₂ is F and the remainder, if any, is selected from -Y, -OY, -SY, -NY₂, wherein Y for -Y is selected from fluorinated or perfluorinated alkyl, alkenyl, alkynyl, aryl, alkylaryl, arylalkyl, alkylene oxide or alkylene imine, and more preferably selected from fluorinated or perfluorinated alkyl; and Y for -OY, -SY, -NY₂ is alkyl, alkenyl, alkynyl or alkylene oxide.

In a preferred embodiment:
- when Y is alkyl, then it is fluorinated or perfluorinated; or particularly
- when Y is alkyl, alkenyl, alkynyl, then it is fluorinated or perfluorinated; or more particularly
- when Y is alkyl, alkenyl, alkynyl, aryl, alkylaryl, arylalkyl, alkylene oxide or alkylene imine, then it is fluorinated or perfluorinated.

In a preferred embodiment:
- when Y in any -Y group is alkyl, then it is fluorinated or perfluorinated; or particularly
- when Y in any -Y group is alkyl, alkenyl, alkynyl, then it is fluorinated or perfluorinated; or more particularly;
- when Y in any -Y group is alkyl, alkenyl, alkynyl, aryl, alkylaryl, arylalkyl, alkylene oxide or alkylene imine, then it is fluorinated or perfluorinated.

In an embodiment, in the compound of formula IV as defined above, Y groups are optionally substituted. In another embodiment, they are substituted. Substitution is preferably at any aliphatic or aromatic C-H bond present in the organic radical.

In a specific embodiment, each Y independently represents an alkyl radical as described above which is fluorinated or perfluorinated, more preferably an alkyl radical comprising from 1 to 4 carbon atoms which is fluorinated or perfluorinated, even more preferably Y represents CF₃.

In a more specific embodiment, at least one of R₁ and R₂ is F and Y at any -Y group at the remainder of R₁ and R₂ is selected from an alkyl radical which is fluorinated or perfluorinated, more preferably an alkyl radical comprising from 1 to 4 carbon atoms which is fluorinated or perfluorinated, even more preferably Y represents CF₃.

In a more specific embodiment, one of R₁ and R₂ is F and the remainder of R₁ and R₂ is selected from -Y, wherein Y is an alkyl radical which is fluorinated or perfluorinated, more preferably an alkyl radical comprising from 1 to 4 carbon atoms which is fluorinated or perfluorinated, even more preferably Y represents CF₃.

Examples of perfluorinated radicals Y are -CF₃, -CF₂CF₃, -CF₂(CF₂)ₙCF₃ (n = 1 to 7), - CF(CF₃)₂, -CF₂CF(CF₃)₂, -CF(CF₃)(CF₂)ₙCF₃ (n = 1 to 5), -CF₂CF(CF₃)(CF₂)ₙCF₃ (n = 1 to 4).-CF=CF₂, -CF=CFCF₃, -CF₂CF=CF₂, -CF=C(CF₃)₂, -CF=CFCF₂CF₃, -CF₂CF=CF₂CF₃, - CF₂CF₂CF=CF₂, -C≡CCF₃, -CF₂C≡CF, -C≡CCF₂CF₃, -CF₂CF₂C≡CF, -C(O)CF₃, - C(O)(CF₂)ₙCF₃ (n = 1 to 6), -C(O)C₆F₅, -C(O)C₆F₄CF₃, -C₆F₅, -C₆F₄CF₃, -C₆F₄CF₂CF₃, - C₆F₃(CF₃)CF₃ (all ortho, meta and para isomers), -CF₂C₆F₅, -CF₂CF₂C₆F₅, -CF₂C₆F₄CF₂ (all ortho, meta and para isomers).

In another embodiment, at least one of R¹ and R² is a halogen, preferably F. In another embodiment, R¹ and R² are both a halogen, preferably F.

In another embodiment, the the dianionic intermediate compound of formula (IV) is characterized in that R¹ and R² are the same.

In another embodiment, the dianionic intermediate compound of formula (IV) is characterized in that R¹ is different from R².

In another embodiment, the dianionic intermediate compound of formula (IV) is characterized in that R¹ and R² are the same and Y represents an organic radical selected from alkyl, alkenyl, alkynyl, aryl and alkylaryl radicals, which are partially fluorinated or perfluorinated.

In another embodiment, the dianionic intermediate compound of formula (IV) is characterized in that R¹ and R² are the same and Y is an alkyl radical comprising from 1 to 4 carbon atoms which is partially fluorinated or perfluorinated, preferably the alkyl radical is perfluorinated methyl (CF₃).

In a most preferred embodiment, the dianionic intermediate compound of formula (IV) is:

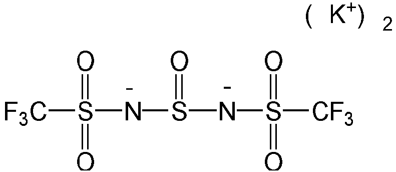

The dianionic intermediate compound of formula (IV) is a versatile synthon that can be used in electrophilic oxidation and oxidative addition reactions.

The invention also refers to a dianion of formula: 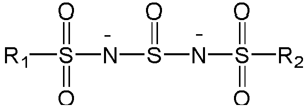 wherein R₁ and R₂ are as defined above for the compound of formula (IV).

### Examples

### Example 1: Preparation of lithium salt (LisFTFSI).

The structures of salt 1 (LisFTFSI) is shown below:

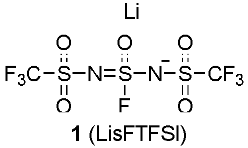

All reagents for the synthesis of (S-fluoro-N-((trifluoromethyl)sulfonyl)sulfonimidoyl)((trifluoromethyl)sulfonyl)amide (LisFTFSI) were purchased from Sigma-Aldrich and used as received. Solvents were distilled and dried prior to use. Specifically, LisFTFSI was prepared in a one-pot fashion by reaction of a sulphonamide salt with an N-(sulfinyl)sulfonamide and subsequent oxidation with an electrophilic fluorine source. More specifically, the synthetic route to lithium (S-fluoro-N-((trifluoromethyl)sulfonyl)sulfonimidoyl)((trifluoromethyl)sulfonyl)amide (LisFTFSI) is illustrated in the scheme below.

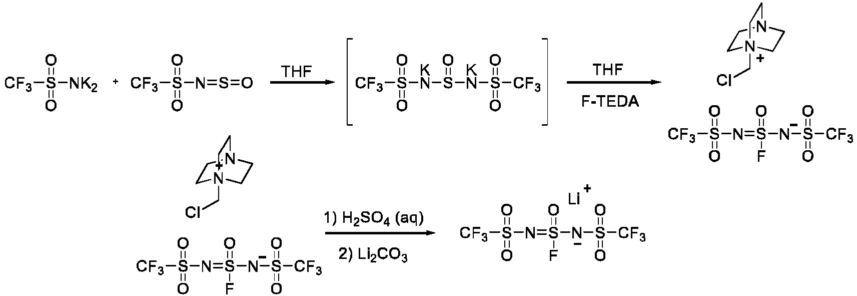

Trifluoromethanesulfonylamide dipotassium salt was obtained by reacting potassium tert-butoxide, KOtBu (7.6 g, 2.5 eq., 68.1 mmol) with trifluoromethanesulfonamide (4.8 g, 32.4 mmol) in THF (70 mL) under reflux for 16 h. The obtained white solid was filtered and washed with abundant THF to yield pure dipotassium salt.

A solution of N-(sulfinyl)trifluoromethanesulfonamide (1 eq, 1.8 mmol), obtained as described in Journal of Fluorine Chemistry, 60 (1993) 283-288, in THF (2.2 mL) was added to a suspension of trifluoromethanesulfonylamide dipotassium salt (0.69 g, 1.5 eq, 2.7 mmol) in THF (2.2 mL) at -20°C. The mixture was stirred and allowed to reach room temperature until complete conversion was achieved as confirmed by ¹⁹F-NMR. A mixture of THF:HFIP (hexafluoro-2-propanol) 6.5:1 (12.1 mL) was added followed by 1-chloromethyl-4-fluoro-1,4-diazoniabicyclo[2.2.2]octane bis(tetrafluoroborate) (1.5 eq, 2.7 mmol), also called F-TEDA. The reaction mixture was stirred until no further evolution was observed by ¹⁹F-NMR. Then, the solvent was removed under reduced pressure to yield an oil. This oil was taken into water (10 mL) and acidified with H₂SO₄. The aqueous solution was extracted with diethyl ether (3x15 mL), organic fractions were combined, and solvent was removed under reduced pressure. An aqueous solution of the obtained acid, N,N'-bis((trifluoromethyl)sulfonyl)sulfuramidimidoyl fluoride, was treated with lithium carbonate (0.07 g, 0.5 eq., 0.9 mmol) and the reaction mixture stirred for 16 h at room temperature. Then solvent was removed in vacuo to yield a white solid. Excess of lithium carbonate was removed by selective dissolution of impurities and recrystallization from acetonitrile. Solvent was removed in vacuo to yield LisFTFSI as a white powder (yield: 4.7 g, 12.9 mmol). ¹⁹F-NMR (283 MHz, MeCN-d3) δ (ppm) 69.18-69.04 (m, 1F, F), -79.36 (d, J = 3.2 Hz, 6F, 2x CF₃). ¹³C-NMR (75 MHz, MeCN-d3): δ (ppm) 121.7 (qd, J = 320.1 Hz, 2.4 Hz, 2x CF₃).

Following the general procedure the Lithium (S-fluoro-N-((trifluoromethyl)sulfonyl)sulfonimidoyl)((trifluoromethyl)sulfonyl)amide (LisFTFSI) was synthesized starting from N-(sulfinyl)trifluoromethanesulfonamide (0.39 g, 1.8 mmol) and using 1-chloromethyl-4-fluoro-1,4-diazoniabicyclo[2.2.2]octane bis(tetrafluoroborate) (0.96 g, 2.7 mmol), also called F-TEDA, as fluorinating reagent. ¹⁹F-NMR (283 MHz, MeCN-d3) δ (ppm) 69.2-69.0 (m, 1F, F), -79.4 (d, J = 3.2 Hz, 6F, 2x CF3). ¹³C-NMR (75 MHz, MeCN-d3): δ (ppm) 121.7 (qd, J = 320.1 Hz, 2.4 Hz, 2x CF3).

### Example 2:

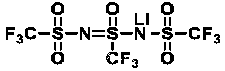

### Lithium (S-trifluoromethyl-N-((trifluoromethyl)sulfonyl)sulfonimidoyl)((trifluoromethyl)sulfonyl)amide

Following the general procedure in example 1, the lithium (S-trifluoromethyl-N-((trifluoromethyl)sulfonyl)sulfonimidoyl)((trifluoromethyl)sulfonyl)amide was synthesized starting from N-(sulfinyl)trifluoromethanesulfonamide (0.39 g, 1.8 mmol) and using 1-Trifluoromethyl-1,2-benziodoxol-3-(1H)-one (0.85 g, 2.7 mmol), also called Togni reagent II, as trifluoromethylating agent. ¹⁹F-NMR (283 MHz, MeCN-d3) δ (ppm) -79.8 (q, *J =* 1.3 Hz, 3F, CF₃), -80.2 (m, 6F, CF₃)

### Example 3:

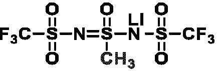

### Lithium (S-methyl-N-((trifluoromethyl)sulfonyl)sulfonimidoyl)((trifluoromethyl)sulfonyl)amide

Following the general procedure above, lithium (S-methyl-N-((trifluoromethyl)sulfonyl)sulfonimidoyl)((trifluoromethyl)sulfonyl)amide was synthesized starting from N-(sulfinyl)trifluoromethanesulfonamide (0.39 g, 1.8 mmol) and using trimethyloxonium tetrafluoroborate (0,40 g, 2.7 mmol) as methylating agent. ¹H-NMR (300 MHz, MeCN-d3) δ (ppm) 3.28 (s, 3H, CH₃) ¹⁹F-NMR (283 MHz, MeCN-d3) δ (ppm) -78.7 (s, 3F, CF₃).

### Example 4:

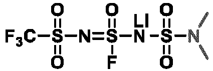

### Lithium (N,N-dimethylsulfamoyl)(S-fluoro-N-((trifluoromethyl)sulfonyl)sulfonimidoyl)amide

Following the general procedure above, lithium (S-fluoro-N-((trifluoromethyl)sulfonyl)sulfonimidoyl)((trifluoromethyl)sulfonyl)amide (LisFTFSI) was synthesized starting from N-(sulfinyl) N,N-dimethylsulfonamide (0.31 g, 1.8 mmol) prepared according to known procedures, i.e. J. Chem. Soc., Perkin Trams. 1, 2002, 1887-1889 and references therein - and using commercial 1-chloromethyl-4-fluoro-1,4-diazoniabicyclo[2.2.2]octane bis(tetrafluoroborate) (0.96 g, 2.7 mmol), also called F-TEDA, as fluorinating reagent. ¹H-NMR (300 MHz, MeCN-d3) δ (ppm) 2.81 (s, 6H, CH₃) ¹⁹F-NMR (283 MHz, MeCN-d3) δ (ppm) 69.1-69.0 (m, 1F, F), -79.2 (d, *J =* 3.2 Hz, 3F, CF₃).

### Example 5:

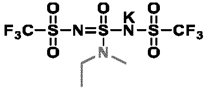

Potassium (N-ethyl-N-methyl-N'-((trifluoromethyl)sulfonyl)sulfamidimidoyl)((trifluoromethyl)sulfonyl)amide was obtained as illustrated in the scheme below by the reaction of KsFTFSI with a nucleophilic amine.

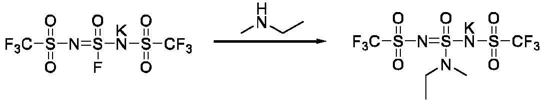

To a solution of KsFTFSI (0.1 g, 0.25 mmol) in toluene (3 mL), N-ethylmethylamine (0.05 mL, 1.5 eq, 0.5 mmol) was added at room temperature. The reaction mixture was heated to reflux for 16 hours. After the reaction was completed, the solvent was removed under reduced pressure and the crude was taken into concentrated HCl (aq.). The aqueous phase was extracted with tert-butyl methyl ether (TBME) (3x15 mL), organic fractions were combined, and solvent was removed under reduced pressure. An aqueous solution of the obtained acid, N-((ethyl(methyl)amino)(oxo)((trifluoromethyl)sulfonamido)-λ⁶-sulfaneylidene)-1,1,1-trifluoromethanesulfonamide was treated with potassium carbonate (0.02 g, 0.5 eq., 0.13 mmol) and the reaction mixture stirred for 16 h at room temperature. Then solvent was removed in vacuo to yield a white solid. Excess of potassium carbonate was removed by selective dissolution of impurities and recrystallization from acetonitrile. Solvent was removed in vacuo to yield a white-off powder. ¹H-NMR (300 MHz, MeCN-d3) δ (ppm) 3.25 (q, *J =* 7.2 Hz, 2H, CH₂), 2.84 (s, 3H, CH₃), 1.19 (t, *J* = 7.2 Hz, 3H, CH₃) ¹⁹F-NMR (283 MHz, MeCN-d3) δ (ppm) -79.2 (s, 3F, CF₃).

## Claims

1. A method to obtain a salt of formula I: 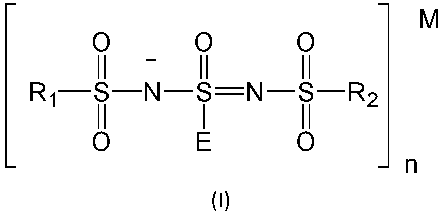 wherein:
- E is a group selected from F; Cl; Br; I; -N(R₄)(R₅), wherein R₄ and R₅ are independently selected from H, an alkyl and an ethylene oxide chain having from 1 to 5 repeating units of ethylene oxide; an alkyl optionally substituted with at least one F, an alkenyl, an aryl, -N(R₆)(R₇) or OR₈, wherein R₆, R₇ and R₈ are independently selected from H and alkyl; and an arylalkenyl;
- R₁ and R₂ are independently selected from
- halogen;
- -Y, wherein Y represents:
- an organic radical selected from alkyl, alkenyl, alkynyl, aryl, alkylaryl, arylalkyl, alkylene oxide or alkylene imine, optionally substituted with at least a substituent selected from the group consisting of F, Cl, Br, I, -CN, -OR', -SR', -NR'₂, wherein R' is H, alkyl, alkylene oxide or alkylene imine; or
- a polymeric group comprising repeating units selected from alkylene oxide, alkylene imine, acrylate, maleimide, phosphazene, siloxane, vinyl alcohol, vinyl amine or mixtures thereof; and
- -OY, -SY, -NY₂, wherein Y represents:
- H or an organic radical selected from alkyl, alkenyl, alkynyl, aryl, alkylaryl, arylalkyl, alkylene oxide or alkylene imine, optionally substituted with at least a substituent selected from the group consisting of F, Cl, Br, I, -CN, -OR', -SR', -NR'₂, wherein R' is H, alkyl, alkylene oxide or alkylene imine; or
- a polymeric group comprising repeating units selected from alkylene oxide, alkylene imine, acrylate, maleimide, phosphazene, siloxane, vinyl alcohol, vinyl amine or mixtures thereof;
wherein each of the two specific substituents Y at NY₂ and R' at NR'₂ is selected independently from the other,
- M is:
- a monovalent, divalent or trivalent cation, selected from ions of alkali metals, of alkaline earth metals, of transition metals and of rare-earth metals;
- an organic onium or polyonium cation; or
- an organometallic cation;
- n is an integer positive number;
said method comprising the steps of:
a) reacting a N-(sulfinyl)sulfonamide of formula (II): 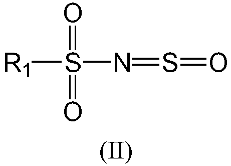 wherein R¹ is as defined above;
with a sulphonamide salt of formula (III): 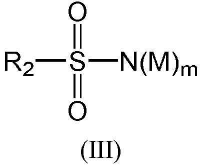 wherein
- R₂ is as defined above,
- M is a proton, or a monovalent or divalent cation selected from ions of alkali metals, of alkaline earth metals, of transition metals, of rare-earth metals, an organic onium cation and an organometallic cation;
- m is 1 if M is a divalent cation, or 2 if M is a proton or a monovalent cation,
provided that when M is a proton, step a) is carried out in presence of a base,
to obtain a dianionic sulfinate intermediate of formula (IV): 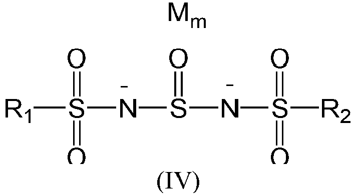
wherein
- R₁, R₂ are as defined above;
- M is a monovalent or divalent cation, selected from ions of alkali metals, of alkaline earth metals, of transition metals, of rare-earth metals, an organic onium cation, and an organometallic cation; and
- m is 1 if M is a divalent cation, or 2 if M is a monovalent cation;
b) oxidizing the sulfinate of formula (IV) with a reagent selected from a fluorination reagent, a chlorination reagent, a bromination reagent, a iodination reagent, an alkylation reagent, and an amination reagent,
to give a salt of formula (I), wherein:
- R₁, R₂ and E are as defined above;
- M is a is a monovalent or a divalent cation, selected from ions of alkali metals, of alkaline earth metals, of transition metals, of rare-earth metals, an organic onium, and an organometallic cation; and
- n is 1 or 2 and corresponds to the valency of M; and
c) carrying out a cation exchange reaction to replace the monovalent or divalent cation M of the intermediate of formula (I) resulting from step b) by a trivalent or a polyonium cation if a salt of formula (I) where M is a trivalent cation or a polyonium cation is to be obtained.

2. The method according to claim 1 wherein M in the compound of formula (I) is:
- a monovalent or divalent cation, selected from ions of alkali metals, of alkaline earth metals, of transition metals and of rare-earth metals;
- an organic onium;
- an organometallic cation.

3. The method according to claim 1 or 2, wherein M is a metal cation selected from K⁺, Li⁺, Na⁺, Cs⁺, Mg²⁺, Ca²⁺, Ba²⁺, Cu²⁺, Zn²⁺, Fe²⁺.

4. The method according to claim 3, wherein M is K⁺.

5. The method according to claim 1 or 2, wherein M in the compound of formula (I) is a polyonium cation selected from polyammonium, polypyrrolidonium, polyimidazolium, polyimidazolinium cation, preferably a polyammonium, polyimidazolium, or polyimidazolinium cation.

6. The method according to any of the preceding claims, wherein E is selected from F, an alkyl substituted with at least one F, and N(R₄)(R₅), wherein R₄ and R₅ are independently selected from a ethylene oxide chain from 1 to 5 repeating units of ethylene oxide.

7. The method according to any of the preceding claims, wherein R₁ and R₂ are independently selected from -Y and -NY₂.

8. The method according to any of the preceding claims, wherein Y represents an organic radical selected from alkyl, alkenyl, alkynyl, aryl and alkylaryl, which are perfluorinated or partially fluorinated.

9. The method according to any of the preceding claims, wherein Y is an alkyl radical comprising from 1 to 4 carbon atoms which is perfluorinated or partially fluorinated.

10. The method according to any of the preceding claims, wherein the reagent used in step b) is selected from 1-Chloromethyl-4-fluoro-1,4-diazoniabicyclo[2.2.2]octane bis(tetrafluoroborate), N-Fluorobenzenesulfonimide (NFSI), N-fluoropyridinium triflate, N-Bromosuccinimide (NBS), N-Chlorosuccinimide (NCS), N-Iodosuccinimide (NIS); iodoalkanes, alkyl triflates, trimethyloxonium tetrafluoroborate, alkyl bromides, Togni reagents (I and II), Langlois reagent (CF3SO₂Na), Umemoto's reagent and chloramines of formula ClNR₄R₅ where R₄ and R₅ are independently selected as defined in claim 1.

11. The method according to claim 10 wherein the reagent used in the step b) is selected from 1-Chloromethyl-4-fluoro-1,4-diazoniabicyclo[2.2.2]octane bis(tetrafluoroborate) and a chloramine of formula ClNR₄R₅ where R₄ and R₅ are independently selected as defined in claim 1.

12. The method according to any of the preceding claims, wherein steps a) and b) are performed in a one-pot manner.

13. A dianionic sulfinate intermediate of formula (IV): 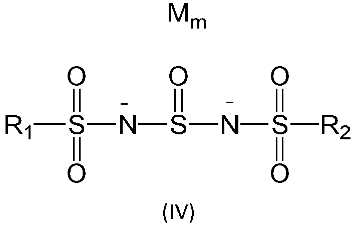 wherein:
- R₁ and R₂ are independently selected from
- halogen;
- -Y, wherein Y represents:
- an organic radical chosen from alkyl, alkenyl, alkynyl, aryl, alkylaryl, arylalkyl, alkylene oxide or alkylene imine, optionally substituted with at least a substituent selected from the group consisting of F, Cl, Br, I, -CN, -OR', -SR', -NR'₂, wherein R' is H, alkyl, alkylene oxide or alkylene imine; or
- a polymeric group comprising repeating units selected from alkylene oxide, alkylene imine, acrylate, maleimide, phosphazene, siloxane, vinyl alcohol, vinyl amine or mixtures thereof; and
- -OY, -SY, -NY₂, wherein Y represents:
- H or an organic radical selected from alkyl, alkenyl, alkynyl, aryl, alkylaryl, arylalkyl, alkylene oxide or alkylene imine, optionally substituted with at least a substituent selected from the group consisting of F, Cl, Br, I, -CN, -OR', -SR', -NR'₂, wherein R' is H, alkyl, alkylene oxide or alkylene imine; or
- a polymeric group comprising repeating units selected from alkylene oxide, alkylene imine, acrylate, maleimide, phosphazene, siloxane, vinyl alcohol, vinyl amine or mixtures thereof;
wherein each of the two specific substituents Y at NY₂ and R' at NR'₂ is selected independently from the other,
and
- M is:
- a monovalent or divalent cation, selected from ions of alkali metals, of alkaline earth metals, of transition metals and of rare-earth metals;
- an organic onium cation; or
- an organometallic cation;
- m is 1 if M is a divalent cation or 2 if M is a monovalent cation.

14. The dianionic sulfinate intermediate according to claim 13, wherein M is a metal cation selected from K⁺, Li⁺, Na⁺, Cs⁺.

15. The dianionic sulfinate intermediate according to any of claims 13 and 14, wherein R₁ and R₂ are independently selected from -Y and -NY₂ and Y represents an organic radical selected from alkyl, alkenyl, alkynyl, aryl and alkylaryl radicals, which are perfluorinated or partially fluorinated.

## Patentansprüche

1. Ein Verfahren, um ein Salz der Formel I zu erhalten: 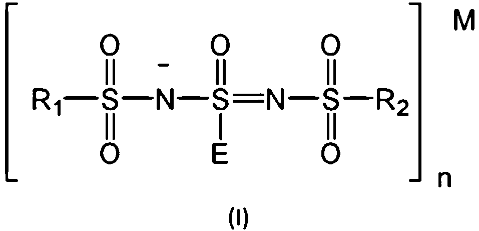 wobei:
- E eine Gruppe ist, ausgewählt aus F; Cl; Br; I; -N(R₄)(R₅), wobei R₄ und R₅ unabhängig voneinander ausgewählt sind aus H, einem Alkyl und einer Ethylenoxidkette mit 1 bis 5 sich wiederholenden Ethylenoxideinheiten; einem Alkyl, das optional mit mindestens einem F, einem Alkenyl, einem Aryl, -N(R₆)(R₇) oder OR₈ substituiert ist, wobei R₆, R₇ und R₈ unabhängig voneinander aus H und Alkyl ausgewählt sind; und einem Arylalkenyl;
- R₁ und R₂ unabhängig voneinander ausgewählt sind aus
- Halogen;
- -Y, wobei Y repräsentiert:
- einen organischen Rest, ausgewählt aus Alkyl, Alkenyl, Alkinyl, Aryl, Alkylaryl, Arylalkyl, Alkylenoxid oder Alkylenimin, optional substituiert mit mindestens einem Substituenten, ausgewählt aus der Gruppe bestehend aus F, Cl, Br, I, -CN, -OR', -SR', -NR'₂, wobei R' H, Alkyl, Alkylenoxid oder Alkylenimin ist;
oder
- eine Polymergruppe, die sich wiederholende Einheiten umfasst, die aus Alkylenoxid, Alkylenimin, Acrylat, Maleimid, Phosphazen, Siloxan, Vinylalkohol, Vinylamin oder Mischungen davon ausgewählt sind; und
- -OY, -SY, -NY₂, wobei Y repräsentiert:
- H oder einen organischen Rest, ausgewählt aus Alkyl, Alkenyl, Alkinyl, Aryl, Alkylaryl, Arylalkyl, Alkylenoxid oder Alkylenimin, optional substituiert mit mindestens einem Substituenten, ausgewählt aus der Gruppe bestehend aus F, Cl, Br, I, -CN, -OR', -SR', -NR'₂, wobei R' H, Alkyl, Alkylenoxid oder Alkylenimin ist; oder
- eine Polymergruppe, die sich wiederholende Einheiten umfasst, die aus Alkylenoxid, Alkylenimin, Acrylat, Maleimid, Phosphazen, Siloxan, Vinylalkohol, Vinylamin oder Mischungen davon ausgewählt sind;
wobei jeder der zwei spezifischen Substituenten Y bei NY₂ und R' bei NR'₂ unabhängig voneinander ausgewählt ist,
- Mist:
- ein einwertiges, zweiwertiges oder dreiwertiges Kation, ausgewählt aus Ionen von Alkalimetallen, Erdalkalimetallen, Übergangsmetallen und Seltenerdmetallen;
- ein organisches Onium- oder Polyonium-Kation; oder
- ein metallorganisches Kation;
- n eine positive ganze Zahl ist;
das Verfahren die folgenden Schritte umfasst:
a) Reagieren eines N-(Sulfinyl)sulfonamids der Formel (II): 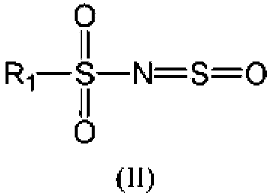 wobei R₁ wie oben definiert ist;
mit einem Sulfonamidsalz der Formel (III): 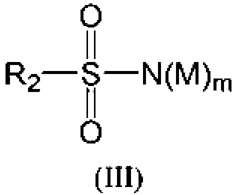
wobei
- R₂ wie oben definiert ist,
- M ein Proton oder ein einwertiges oder zweiwertiges Kation ist, ausgewählt aus Ionen von Alkalimetallen, Erdalkalimetallen, Übergangsmetallen, Seltenerdmetallen, einem organischen Oniumkation und einem organometallischen Kation;
- m 1 ist, wenn M ein zweiwertiges Kation ist, oder 2, wenn M ein Proton oder ein einwertiges Kation ist,
vorausgesetzt, dass, wenn M ein Proton ist, Schritt a) in Gegenwart einer Base durchgeführt wird,
um ein dianionisches Sulfinat-Zwischenprodukt der Formel (IV) zu erhalten: 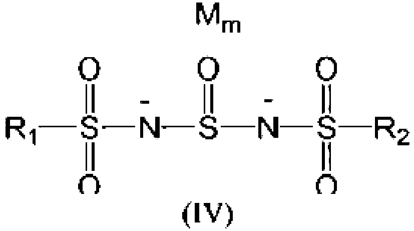
wobei
- R₁, R₂ wie oben definiert sind;
- M ein einwertiges oder zweiwertiges Kation, ausgewählt aus Ionen von Alkalimetallen, Erdalkalimetallen, Übergangsmetallen, Seltenerdmetallen, einem organischen Oniumkation und einem organometallischen Kation ist; und
- m 1 ist, wenn M ein zweiwertiges Kation ist, oder 2, wenn M ein einwertiges Kation ist;
b) Oxidieren des Sulfinats der Formel (IV) mit einem Reagenz, das aus einem Fluorierungsreagenz, einem Chlorierungsreagenz, einem Bromierungsreagenz, einem lodierungsreagenz, einem Alkylierungsreagenz und einem Aminierungsreagenz ausgewählt ist, um ein Salz der Formel (I) zu erhalten, wobei:
- R₁, R₂ und E wie oben definiert sind;
- M ein einwertiges oder zweiwertiges Kation, ausgewählt aus Ionen von Alkalimetallen, Erdalkalimetallen, Übergangsmetallen, Seltenerdmetallen, einem organischen Onium und einem organometallischen Kation, ist; und
- n 1 oder 2 ist und der Valenz von M entspricht; und
c) Durchführung einer Kationenaustauschreaktion, um das einwertige oder zweiwertige Kation M des aus Schritt b) resultierenden Zwischenprodukts der Formel (I) durch ein dreiwertiges oder ein Polyoniumkation zu ersetzen, wenn ein Salz der Formel (I) erhalten werden soll, wobei M ein dreiwertiges Kation oder ein Polyoniumkation ist.

2. Das Verfahren gemäß Anspruch 1, wobei M in der Verbindung der Formel (I)
- ein einwertiges oder zweiwertiges Kation, ausgewählt aus Ionen von Alkalimetallen, Erdalkalimetallen, Übergangsmetallen und Seltenerdmetallen;
- ein organisches Onium;
- ein organometallisches Kation ist.

3. Das Verfahren gemäß Anspruch 1 oder 2, wobei M ein Metallkation ist, ausgewählt aus K⁺, Li⁺, Na⁺, Cs⁺, Mg²⁺, Ca²⁺, Ba²⁺, Cu²⁺, Zn²⁺, Fe²⁺.

4. Das Verfahren gemäß Anspruch 3, wobei M K⁺ ist.

5. Das Verfahren gemäß Anspruch 1 oder 2, wobei M in der Verbindung der Formel (I) ein Polyoniumkation ist, ausgewählt aus Polyammonium-, Polypyrrolidonium-, Polyimidazolium-, Polyimidazolinium-Kationen, bevorzugt ein Polyammonium-, Polyimidazolium- oder Polyimidazolinium-Kation.

6. Das Verfahren gemäß irgendeinem der vorstehenden Ansprüche, wobei E ausgewählt ist aus F, einem Alkyl, das mit mindestens einem F substituiert ist, und N(R₄)(R₅), wobei R₄ und R₅ unabhängig voneinander ausgewählt sind aus einer Ethylenoxidkette mit 1 bis 5 sich wiederholenden Ethylenoxideinheiten.

7. Das Verfahren gemäß irgendeinem der vorstehenden Ansprüche, wobei R₁ und R₂ unabhängig voneinander aus -Y und -NY₂ ausgewählt sind.

8. Das Verfahren gemäß irgendeinem der vorstehenden Ansprüche, wobei Y einen organischen Rest repräsentiert, welcher aus Alkyl, Alkenyl, Alkinyl, Aryl und Alkylaryl ausgewählt ist, die perfluoriert oder teilweise fluoriert sind.

9. Das Verfahren gemäß irgendeinem der vorstehenden Ansprüche, wobei Y ein Alkylrest ist, der 1 bis 4 Kohlenstoffatome umfasst, welcher vollständig oder teilweise fluoriert ist.

10. Das Verfahren gemäß irgendeinem der vorstehenden Ansprüche, wobei das in Schritt b) verwendete Reagenz ausgewählt ist aus 1-Chlormethyl-4-fluor-1,4-diazoniabicyclo[2.2.2]octan-bis(tetrafluorborat), N-Fluorbenzolsulfonimid (NFSI), N-Fluorpyridiniumtriflat, N-Bromsuccinimid (NBS), N-Chlorsuccinimid (NCS), N-lodosuccinimid (NIS); lodalkane, Alkyltriflate, Trimethyloxoniumtetrafluorborat, Alkylbromide, Togni-Reagenzien (I und II), Langlois-Reagenz (CF3SO₂Na), Umemoto-Reagenz und Chloramine der Formel ClNR₄R₅, wobei R₄ und R₅ unabhängig voneinander wie in Anspruch 1 definiert ausgewählt sind.

11. Das Verfahren gemäß Anspruch 10, wobei das in Schritt b) verwendete Reagenz ausgewählt ist aus 1-Chlormethyl-4-fluor-1,4-diazoniabicyclo[2.2.2]octan-bis(tetrafluorborat) und einem Chloramin der Formel ClNR₄R₅, wobei R₄ und R₅ unabhängig voneinander wie in Anspruch 1 definiert ausgewählt sind.

12. Das Verfahren gemäß irgendeinem der vorstehenden Ansprüche, wobei die Schritte a) und b) in einem Ein-Topf-Verfahren durchgeführt werden.

13. Ein dianionisches Sulfinat-Zwischenprodukt der Formel (IV): 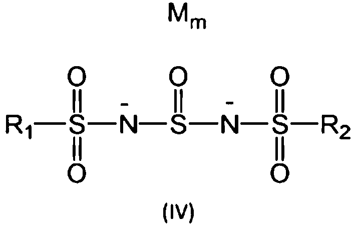 wobei:
- R₁ und R₂ unabhängig voneinander ausgewählt sind aus
- Halogen;
- -Y, wobei Y repräsentiert:
- einen organischen Rest, ausgewählt aus Alkyl, Alkenyl, Alkinyl, Aryl, Alkylaryl, Arylalkyl, Alkylenoxid oder Alkylenimin, optional substituiert mit mindestens einem Substituenten, ausgewählt aus der Gruppe bestehend aus F, Cl, Br, I, -CN, -OR', -SR', -NR'₂, wobei R' H, Alkyl, Alkylenoxid oder Alkylenimin ist;
oder
- eine Polymergruppe, die sich wiederholende Einheiten umfasst, die aus Alkylenoxid, Alkylenimin, Acrylat, Maleimid, Phosphazen, Siloxan, Vinylalkohol, Vinylamin oder Mischungen davon ausgewählt sind; und
- -OY, -SY, -NY₂, wobei Y repräsentiert:
- H oder einen organischen Rest, ausgewählt aus Alkyl, Alkenyl, Alkinyl, Aryl, Alkylaryl, Arylalkyl, Alkylenoxid oder Alkylenimin, optional substituiert mit mindestens einem Substituenten, ausgewählt aus der Gruppe bestehend aus F, Cl, Br, I, -CN, -OR', -SR', -NR'₂, wobei R' H, Alkyl, Alkylenoxid oder Alkylenimin ist; oder
- eine Polymergruppe, die sich wiederholende Einheiten umfasst, die aus Alkylenoxid, Alkylenimin, Acrylat, Maleimid, Phosphazen, Siloxan, Vinylalkohol, Vinylamin oder Mischungen davon ausgewählt sind;
wobei jeder der zwei spezifischen Substituenten Y bei NY₂ und R' bei NR'₂ unabhängig voneinander ausgewählt ist,
und
- M ist:
- ein einwertiges oder zweiwertiges Kation, ausgewählt aus Ionen von Alkalimetallen, Erdalkalimetallen, Übergangsmetallen und Seltenerdmetallen; oder
- ein metallorganisches Kation;
- m 1 ist, wenn M ein zweiwertiges Kation ist, oder 2, wenn M ein einwertiges Kation ist.

14. Das dianionische Sulfinat-Zwischenprodukt gemäß Anspruch 13, wobei M ein Metallkation ist, ausgewählt aus K⁺, Li⁺, Na⁺, Cs⁺.

15. Das dianionische Sulfinat-Zwischenprodukt nach irgendeinem der Ansprüche 13 und 14, wobei R₁ und R₂ unabhängig voneinander aus -Y und -NY₂ ausgewählt sind und Y einen organischen Rest repräsentiert, welcher aus Alkyl-, Alkenyl-, Alkinyl-, Aryl- und Alkylarylresten ausgewählt ist, die perfluoriert oder teilweise fluoriert sind.

## Revendications

1. Méthode pour obtenir un sel de formule I: 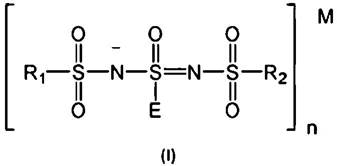 dans laquelle :
- E est un groupe sélectionné parmi F; CI; Br; I; -N(R₄)(R₅), où R₄ et R₅ sont indépendamment choisis parmi H, un groupe alkyle et une chaîne d'oxide d'éthylène ayant de 1 à 5 unités répétitives d'oxide d'éthylène ; un groupe alkyle éventuellement substitué par au moins un F, un groupe alcényle, un groupe aryle, -N(R₆)(R₇) ou OR₈, dans lequel R₆, R₇ et R₈ sont indépendamment choisis parmi H et un groupe alkyle ; et un groupe arylalcényle ;
- R₁ et R₂ sont indépendamment choisis parmi
- halogène ;
- -Y, où Y représente :
- un radical organique choisi parmi les groupes alkyle, alcényle, alcynyle, aryle, alkylaryle, arylalkyle, oxyde d'alkylène ou alkylène imine, éventuellement substitué par au moins un substituant choisi dans le groupe constitué par F, CI, Br, I, -CN, -OR', -SR', -NR'₂, dans lequel R' est H, un groupe alkyle, un groupe oxyde d'alkylène ou alkylène imine ; ou
- un groupe polymère comprenant des unités répétitives choisies parmi l'oxyde d'alkylène, l'imine d'alkylène, l'acrylate, le maléimide, le phosphazène, le siloxane, l'alcool vinylique, la vinylamine ou leurs mélanges ; et
- -OY, -SY, -NY₂, où Y représente :
- H ou un radical organique choisi parmi les groupes alkyle, alcényle, alcynyle, aryle, alkylaryle, arylalkyle, oxyde d'alkylène ou alkylène imine, éventuellement substitué par au moins un substituant choisi dans le groupe constitué par F, CI, Br, 1, -CN, -OR', -SR', -NR'₂, dans lequel R' is H, un groupe alkyle, un groupe oxyde d'alkylène ou alkylène imine ; ou
- un groupe polymère comprenant des unités répétitives choisies parmi l'oxyde d'alkylène, l'alkylène imine, l'acrylate, le maléimide, le phosphazène, le siloxane, l'alcool vinylique, la vinylamine ou leurs mélanges ;
dans lequel chacun des deux substituants Y de NY₂ et R' de NR'₂ est choisi indépendamment l'un de l'autre,
- M est :
- un cation monovalent, divalent ou trivalent, choisi parmi les ions métalliques alcalins, de métaux alcalino-terreux, de métaux de transition et de métaux des terres rares ;
- un cation organique onium ou polyonium ; ou
- un cation organométallique ;
- n est un nombre entier positif ;
ladite méthode comprenant les étapes de :
a) faire réagir un N-(sulfinyl)sulfonamide de formule (II) : 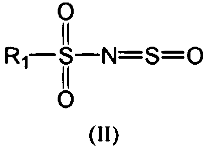 dans laquelle R¹ est tel que défini ci-dessus ;
avec un sel de sulphonamide de formule (III) : 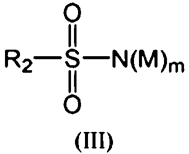 dans laquelle
- R₂ est tel que défini ci-dessus,
- M est un proton, ou un cation monovalent ou divalent choisi parmi les ions métalliques alcalins, de métaux alcalino-terreux, de métaux de transition, de métaux des terres rares, un cation onium organique et un cation organométallique ;
- m est égal à 1 si M est un cation divalent, ou à 2 si M est un proton ou un cation monovalent,
à condition que, lorsque M est un proton, l'étape a) soit réalisée en présence d'une base,
pour obtenir un intermédiaire sulfinate dianionique de formule (IV): 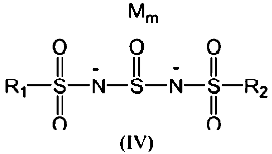 dans laquelle
- R₁, R₂ sont tels que définis ci-dessus;
- M est un cation monovalent ou divalent, choisi parmi les ions métalliques alcalins, de métaux alcalino-terreux, de métaux de transition, de métaux des terres rares, un cation onium organique et un cation organométallique ; et
- m est égal à 1 si M est un cation divalent, ou à 2 si M est un cation monovalent ;
b) oxyder le sulfinate de formule (IV) avec un réactif choisi parmi un réactif de fluoration, un réactif de chloration, un réactif de bromation, un réactif d'iodation, un réactif d'alkylation et un réactif d'amination,
pour obtenir un sel de formule (I), dans laquelle :
- R₁, R₂ et E sont tels que définis ci-dessus ;
- M représente un cation monovalent ou divalent, choisi parmi les ions métalliques alcalins, de métaux alcalino-terreux, de métaux de transition, de métaux des terres rares, un onium organique et un cation organométallique ; et
- n est égal à 1 ou 2 et correspond à la valence de M ; et
c) effectuer une réaction d'échange cationique pour remplacer le cation monovalent ou divalent M de l'intermédiaire de formule (I) issu de l'étape b) par un cation trivalent ou polyonium si l'on souhaite obtenir un sel de formule (I) où M est un cation trivalent ou polyonium.

2. La méthode selon la revendication 1, dans lequel M dans le composé de formule (I) est :
- un cation monovalent ou divalent, choisi parmi les ions métalliques alcalins, de métaux alcalino-terreux, de métaux de transition et de métaux des terres rares ;
- un onium organique ;
- un cation organométallique.

3. La méthode selon la revendication 1 ou 2, dans lequel M est un cation métallique choisi parmi K⁺, Li⁺, Na⁺, Cs⁺, Mg²⁺, Ca²⁺, Ba²⁺, Cu²⁺, Zn²⁺, Fe²⁺.

4. La méthode selon la revendication 3, dans lequel M est K⁺.

5. La méthode selon la revendication 1 ou 2, dans lequel M du composé de formule (I) est un cation polyonium choisi parmi les cations polyammonium, polypyrrolidonium, polyimidazolium ou polyimidazolinium, de préférence un cation polyammonium, polyimidazolium ou polyimidazolinium.

6. La méthode selon l'une quelconque des revendications précédentes, dans laquelle E est choisi parmi F, un groupe alkyle substitué par au moins un groupe F, et N(R₄)(R₅), où R₄ et R₅ sont indépendamment choisis parmi une chaîne d'oxyde d'éthylène de 1 à 5 unités répétitives d'oxyde d'éthylène.

7. La méthode selon l'une quelconque des revendications précédentes, dans laquelle R₁ et R₂ sont indépendamment choisis parmi -Y et -NY₂.

8. La méthode selon l'une quelconque des revendications précédentes, dans laquelle Y représente un radical organique choisi parmi les groupes alkyle, alcényle, alcynyle, aryle et alkylaryle, perfluorés ou partiellement fluorés.

9. La méthode selon l'une quelconque des revendications précédentes, dans laquelle Y est un radical alkyle comprenant de 1 à 4 atomes de carbone, perfluoré ou partiellement fluoré.

10. La méthode selon l'une quelconque des revendications précédentes, dans laquelle le réactif utilisé à l'étape b) est choisi parmi le bis(tétrafluoroborate) de I-chlorométhyl-4-fluoro-1,4-diazoniabicyclo[2.2.2]octane, le N-fluorobenzènesulfonimide (NFSI), le triflate de N-fluoropyridinium, le N bromosuccinimide (NBS), le N-chlorosuccinimide (NCS), le N-iodosuccinimide (NIS); les iodoalcanes, les triflates d'alkyle, le tétrafluoroborate de triméthyloxonium, les bromures d'alkyle, les réactifs de Togni (I et II), le réactif de Langlois (CF₃SO₂Na), le réactif d'Umemoto et les chloramines de formule ClNR₄R₅ où R₄ et R₅ sont indépendamment choisis tels que définis dans la revendication 1.

11. La méthode selon la revendication 10, dans laquelle le réactif utilisé à l'étape b) est choisi parmi le bis(tétrafluoroborate) de 1-chlorométhyl-4-fluoro-1,4-diazoniabicyclo[2.2.2]octane et une chloramine de formule ClNR₄R₅, où R4 et R5 sont indépendamment choisis comme défini dans la revendication 1.

12. La méthode selon l'une quelconque des revendications précédentes, dans laquelle les étapes a) et b) sont réalisées dans un seul pot.

13. Intermédiaire sulfinate dianionique de formule (IV) : 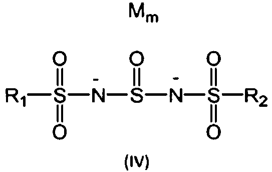 dans lequel:
- R₁ et R₂ sont indépendamment choisis parmi
- halogène ;
- -Y, où Y représente :
- un radical organique choisi parmi les groupes alkyle, alcényle, alcynyle, aryle, alkylaryle, arylalkyle, oxyde d'alkylène ou alkylène imine, éventuellement substitué par au moins un substituant choisi dans le groupe constitué par F, CI, Br, I, -CN, -OR', -SR', -NR'₂, où R' représente H, un groupe alkyle, oxyde d'alkylène ou alkylène imine ; ou
- un groupe polymère comprenant des motifs répétitifs choisis parmi l'oxyde d'alkylène, l'alkylène imine, l'acrylate, le maléimide, le phosphazène, le siloxane, l'alcool vinylique, la vinylamine ou leurs mélanges ; et
- -OY, -SY, -NY₂, où Y représente :
- H ou un radical organique choisi parmi les groupes alkyle, alcényle, alcynyle, aryle, alkylaryle, arylalkyle, oxyde d'alkylène ou alkylène imine, éventuellement substitué par au moins un substituant choisi dans le groupe constitué par F, CI, Br, I, -CN, -OR', -SR', -NR'₂, où R' est H, alkyle, oxyde d'alkylène ou alkylène imine ; ou
- un groupe polymère comprenant des motifs répétitifs choisis parmi l'oxyde d'alkylène, l'alkylène imine, l'acrylate, le maléimide, le phosphazène, le siloxane, l'alcool vinylique, la vinylamine ou leurs mélanges ;
où chacun des deux substituants spécifiques Y de NY₂ et R' de NR'₂ est choisi indépendamment l'un de l'autre,
et
- M est :
- un cation monovalent ou divalent, choisi parmi les ions métalliques alcalins, de métaux alcalino-terreux, de métaux de transition et de métaux des terres rares ;
- un cation onium organique ; ou
- un cation organométallique ;
- m est égal à 1 si M est un cation divalent ou à 2 si M est un cation monovalent.

14. L'intermédiaire sulfinate dianionique selon la revendication 13, dans lequel M est un cation métallique choisi parmi K⁺, Li⁺, Na⁺, Cs⁺.

15. L'intermédiaire sulfinate dianionique selon l'une quelconque des revendications 13 et 14, dans lequel R₁ et R₂ sont indépendamment choisis parmi -Y et -NY₂ et Y représente un radical organique choisi parmi les radicaux alkyle, alcényle, alcynyle, aryle et alkylaryle, qui sont perfluorés ou partiellement fluorés.
